# EUROPEAN PATENT SPECIFICATION

(11) **EP 2 847 348 B1**
(45) Date of publication and mention of the grant of the patent: **04.07.2018**
(21) Application number: 13720525.8
(22) Date of filing: 02.05.2013
(51) Int. Cl.: C12Q 1/68

(54) **A SCREENING METHOD FOR THE DETECTION OF CLOSTRIDIUM DIFFICILE**
SCREENING-VERFAHREN ZUM NACHWEIS VON CLOSTRIDIUM DIFFICILE
PROCÉDÉ DE CRIBLAGE POUR LA DÉTECTION DE CLOSTRIDIUM DIFFICILE

(30) Priority: 08.05.2012 GB 201207998
(43) Date of publication of application: 18.03.2015
(73) Proprietor: University College Cardiff Consultants Limited, South Glamorgan CF24 0DE (GB)
(72) Inventor: BAILLIE, Lesley William James, Hilperton Wiltshire BA14 7TN (GB); JOSHI, Lovleen Tina, Cardiff CF24 2TW (GB)
(74) Representative: Fyles, Julie Marie
(86) International application number: PCT/GB2013/051149
(87) International publication number: WO 2013/167876

(56) References cited:
- WO-A1-2009/061752
- WO-A1-2010/062897
- WO-A1-2010/116290
- WO-A2-91/09973
- US-A1- 2004 265 230
- US-A1- 2012 070 859
- TENNANT SHARON M., ZHANG YONGXIA, GALEN JAMES, GEDDES CHRIS, LEVINE MYRON, CHAKRAVORTTY DIPSHIKHA: "Ultra-Fast and Sensitive Detection of Non-Typhoidal Salmonella Using Microwave-Accelerated Metal-Enhanced Fluorescence (MAMEF)", PLOS ONE, vol. 6, no. 4, E18700, 2011, pages 1-8, XP002698846, cited in the application

### SEQUENCE LISTING

<110> University College Cardiff Consultants Limited
<120> A screening method for the detection of Clostridium difficile
<130> 0092P/WO S1
<150> GB1207998.4
   <151> 2012-05-08
<160> 77
<170> PatentIn version 3.5
<210> 1
   <211> 39
   <212> DNA
   <213> Clostridium difficile
<400> 1
   tcaagactct attatagtaa gtgcaaatca atatgaagt 39
<210> 2
   <211> 76
   <212> DNA
   <213> Clostridium difficile
<400> 2
   aaaatattac tttaatacta acactgctgt tgcagttact ggatggcaaa ctattaatgg 60
   taaaaaatac tacttt 76
<210> 3
   <211> 17
   <212> DNA
   <213> Clostridium difficile
<400> 3
   tactttaata ctaacac 17
<210> 4
   <211> 17
   <212> DNA
   <213> Clostridium difficile
<400> 4
   tttaatacta acactgc 17
<210> 5
   <211> 17
   <212> DNA
   <213> Clostridium difficile
<400> 5
   actaacactg ctgttgc 17
<210> 6
   <211> 17
   <212> DNA
   <213> Clostridium difficile
<400> 6
   tgctgttgca gttactg 17
<210> 7
   <211> 22
   <212> DNA
   <213> Clostridium difficile
<400> 7
   tgctgttgca gttactggat gg 22
<210> 8
   <211> 22
   <212> DNA
   <213> Clostridium difficile
<400> 8
   tgttgcagtt actggatggc aa 22
<210> 9
   <211> 22
   <212> DNA
   <213> Clostridium difficile
<400> 9
   atggcaaact attaatggta aa 22
<210> 10
   <211> 22
   <212> DNA
   <213> Clostridium difficile
<400> 10
   gatggcaaac tattaatggt aa 22
<210> 11
   <211> 17
   <212> DNA
   <213> Clostridium difficile
<400> 11
   tcaagactct attatag 17
<210> 12
   <211> 22
   <212> DNA
   <213> Clostridium difficile
<400> 12
   taagtgcaaa tcaatatgaa gt 22
<210> 13
   <211> 17
   <212> DNA
   <213> Clostridium difficile
<400> 13
   ttaatgccat ctataag 17
<210> 14
   <211> 17
   <212> DNA
   <213> Clostridium difficile
<400> 14
   aatgccatct ataagtc 17
<210> 15
   <211> 17
   <212> DNA
   <213> Clostridium difficile
<400> 15
   gccatctata agtcaag 17
<210> 16
   <211> 17
   <212> DNA
   <213> Clostridium difficile
<400> 16
   catctataag tcaagac 17
<210> 17
   <211> 17
   <212> DNA
   <213> Clostridium difficile
<400> 17
   tctataagtc aagactc 17
<210> 18
   <211> 22
   <212> DNA
   <213> Clostridium difficile
<400> 18
   ctataagtca atatgaagtt ag 22
<210> 19
   <211> 22
   <212> DNA
   <213> Clostridium difficile
<400> 19
   aatatgaagt tagaataaat ag 22
<210> 20
   <211> 22
   <212> DNA
   <213> Clostridium difficile
<400> 20
   tatgaagtta gaataaatag tg 22
<210> 21
   <211> 22
   <212> DNA
   <213> Clostridium difficile
<400> 21
   aagttagaat aaatagtgaa gg 22
<210> 22
   <211> 17
   <212> DNA
   <213> Clostridium difficile
<400> 22
   ctgcgaacta ttgatgg 17
<210> 23
   <211> 17
   <212> DNA
   <213> Clostridium difficile
<400> 23
   atggtaaaaa atattac 17
<210> 24
   <211> 17
   <212> DNA
   <213> Clostridium difficile
<400> 24
   aaatattact ttaatac 17
<210> 25
   <211> 17
   <212> DNA
   <213> Clostridium difficile
<400> 25
   attactttaa tactaac 17
<210> 26
   <211> 22
   <212> DNA
   <213> Clostridium difficile
<400> 26
   gtaaaaaata ttactttaat ac 22
<210> 27
   <211> 22
   <212> DNA
   <213> Clostridium difficile
<400> 27
   aaaatattac tttaatacta ac 22
<210> 28
   <211> 22
   <212> DNA
   <213> Clostridium difficile
<400> 28
   aatattactt taatactaac ac 22
<210> 29
   <211> 22
   <212> DNA
   <213> Clostridium difficile
<400> 29
   attactttaa tactaacact gc 22
<210> 30
   <211> 22
   <212> DNA
   <213> Clostridium difficile
<400> 30
   tactttaata ctaacactgc tg 22
<210> 31
   <211> 22
   <212> DNA
   <213> Clostridium difficile
<400> 31
   ttaatactaa cactgctgtt gc 22
<210> 32
   <211> 22
   <212> DNA
   <213> Clostridium difficile
<400> 32
   aatactaaca ctgctgttgc ag 22
<210> 33
   <211> 22
   <212> DNA
   <213> Clostridium difficile
<400> 33
   ttttttttaa tactaacact gc 22
<210> 34
   <211> 22
   <212> DNA
   <213> Clostridium difficile
<400> 34
   ttttttcaag actctattat ag 22
<210> 35
   <211> 22
   <212> DNA
   <213> Clostridium difficile
<400> 35
   tgttgcagtt actggatggc aa 22
<210> 36
   <211> 21
   <212> DNA
   <213> Clostridium difficile
<400> 36
   taagtgcaaa tcaatatgaa g 21
<210> 37
   <211> 49
   <212> DNA
   <213> Clostridium difficile
<400> 37
   aaattatgat tgtgacgtaa tcccaataca acgtcaatga cctaccgtt 49
<210> 38
   <211> 48
   <212> DNA
   <213> Clostridium difficile
<400> 38
   agttctgaga taatatctaa tcccaatatt cacgtttagt tatacttg 48
<210> 39
   <211> 50
   <212> DNA
   <213> Clostridium difficile
<400> 39
   atggatttga atactttgca cctgctaata cggatgctaa caacatagaa 50
<210> 40
   <211> 51
   <212> DNA
   <213> Clostridium difficile
<400> 40
   ttggcaaata agctatcttt taactttagt gataaacaag atgtacctgt a 51
<210> 41
   <211> 41
   <212> DNA
   <213> Clostridium difficile
<400> 41
   catattctgg tatattaaat ttcaataata aaatttacta t 41
<210> 42
   <211> 52
   <212> DNA
   <213> Clostridium difficile
<400> 42
   tttgagggag aatcaataaa ctatactggt tggttagatt tagatgaaaa ga 52
<210> 43
   <211> 17
   <212> DNA
   <213> Clostridium difficile
<400> 43
   ggatttgaat actttgc 17
<210> 44
   <211> 17
   <212> DNA
   <213> Clostridium difficile
<400> 44
   tttgaatact ttgcacc 17
<210> 45
   <211> 17
   <212> DNA
   <213> Clostridium difficile
<400> 45
   gaatactttg cacctgc 17
<210> 46
   <211> 22
   <212> DNA
   <213> Clostridium difficile
<400> 46
   acctgctaat acggatgcta ac 22
<210> 47
   <211> 22
   <212> DNA
   <213> Clostridium difficile
<400> 47
   tgctaatacg gatgctaaca ac 22
<210> 48
   <211> 22
   <212> DNA
   <213> Clostridium difficile
<400> 48
   taatacggat gctaacaaca ta 22
<210> 49
   <211> 9
   <212> DNA
   <213> Clostridium difficile
<400> 49
   aacatagaa 9
<210> 50
   <211> 6
   <212> DNA
   <213> Clostridium difficile
<400> 50
   atagaa 6
<210> 51
   <211> 3
   <212> DNA
   <213> Clostridium difficile
<400> 51
   gaa 3
<210> 52
   <211> 11
   <212> DNA
   <213> Clostridium difficile
<400> 52
   caaactatta a 11
<210> 53
   <211> 11
   <212> DNA
   <213> Clostridium difficile
<400> 53
   actattaatg g 11
<210> 54
   <211> 10
   <212> DNA
   <213> Clostridium difficile
<400> 54
   aaatacactt 10
<210> 55
   <211> 11
   <212> DNA
   <213> Clostridium difficile
<400> 55
   aaaatactac t 11
<210> 56
   <211> 17
   <212> DNA
   <213> Clostridium difficile
<400> 56
   ttggcaaata agctatc 17
<210> 57
   <211> 17
   <212> DNA
   <213> Clostridium difficile
<400> 57
   ataagctatc ttttaac 17
<210> 58
   <211> 16
   <212> DNA
   <213> Clostridium difficile
<400> 58
   tcttttaact ttagtg 16
<210> 59
   <211> 22
   <212> DNA
   <213> Clostridium difficile
<400> 59
   ttttaacttt agtgataaac aa 22
<210> 60
   <211> 22
   <212> DNA
   <213> Clostridium difficile
<400> 60
   tttagtgata aacaagatgt ac 22
<210> 61
   <211> 20
   <212> DNA
   <213> Clostridium difficile
<400> 61
   ataaacaaga tgtacctgta 20
<210> 62
   <211> 11
   <212> DNA
   <213> Clostridium difficile
<400> 62
   gatgtacctg t 11
<210> 63
   <211> 5
   <212> DNA
   <213> Clostridium difficile
<400> 63
   ctgta 5
<210> 64
   <211> 17
   <212> DNA
   <213> Clostridium difficile
<400> 64
   ctggtatatt aaatttc 17
<210> 65
   <211> 18
   <212> DNA
   <213> Clostridium difficile
<400> 65
   aataataaaa tttactat 18
<210> 66
   <211> 17
   <212> DNA
   <213> Clostridium difficile
<400> 66
   agggagaatc aataaac 17
<210> 67
   <211> 17
   <212> DNA
   <213> Clostridium difficile
<400> 67
   gaatcaataa actatac 17
<210> 68
   <211> 17
   <212> DNA
   <213> Clostridium difficile
<400> 68
   tcaataaact atactgg 17
<210> 69
   <211> 17
   <212> DNA
   <213> Clostridium difficile
<400> 69
   taaactatac tggttgg 17
<210> 70
   <211> 22
   <212> DNA
   <213> Clostridium difficile
<400> 70
   tatactggtt ggttagattt ag 22
<210> 71
   <211> 22
   <212> DNA
   <213> Clostridium difficile
<400> 71
   tggttggtta gatttagatg aa 22
<210> 72
   <211> 22
   <212> DNA
   <213> Clostridium difficile
<400> 72
   ttggttagat ttagatgaaa ag 22
<210> 73
   <211> 22
   <212> DNA
   <213> Clostridium difficile
<400> 73
   ttggttagat ttagatgaaa ag 22
<210> 74
   <211> 19
   <212> DNA
   <213> Clostridium difficile
<400> 74
   ttagatttag atgaaaaga 19
<210> 75
   <211> 10
   <212> DNA
   <213> Clostridium difficile
<400> 75
   atgaaaagaa 10
<210> 76
   <211> 4
   <212> DNA
   <213> Clostridium difficile
<400> 76
   aaga 4
<210> 77
   <211> 1
   <212> DNA
   <213> Clostridium difficile
<400> 77
   a 1

## Description

### Field of the Invention

The invention relates to a screening method for the detection of *Clostridium difficile,* including various strains and toxinotypes thereof, in a sample which method comprises the binding of at least one oligonucleotide probe to a target region; a kit for carrying out said method; an array, comprising at least one oligonucleotide probe, for use in said screening method; and at least one oligonucleotide probe, ideally, for use in said screening method.

### Background of the Invention

Healthcare-associated infections (HCAIs), or nosocomial infections, are those that are acquired by a patient during the course of receiving treatment within a healthcare setting. HCAIs can affect any part of the body, including the urinary system (urinary tract infection), the respiratory system (pneumonia or respiratory tract infection), the skin, surgical wounds (surgical site infection), the gastrointestinal system and even the bloodstream (bacteraemia). Many such infections may arise from the presence of micro-organisms on the body of the patient; however, they may also be caused by micro-organisms originating from another patient or from micro-organisms transmitted from the hospital environment due to poor hygiene conditions.

HCAIs are amongst the major causes of death and increased morbidity in hospitalised patients. Each year, at least 2,000,000 patients in the USA and over 320,000 patients in the UK acquire one or more HCAIs during their stay in hospital. It is predicted that 1 in 4 patients in intensive care worldwide will acquire an infection during their treatment, with this estimate doubled in less developed countries. It has been reported that at any one time more than 1.4 million people worldwide are suffering from an HCAI. In the US, 1 in every 136 patients a day becomes severely ill as a consequence of contracting an HCAI, which equates to the above 2 million cases per year. As a consequence, it is predicted patients spend an average of 2.5 times longer in hospital. In addition to reducing patient safety, HCAIs also constitute a significant financial burden on healthcare systems. In the USA, the risk of HCAIs has risen steadily over the last decade with accompanying costs estimated at US$ 4.5-5.7 billion a year. Similarly in the UK, HCAIs are estimated to cost the NHS £1 billion a year.

The micro-organisms giving rise to HCAIs are numerous, and may be in the form of any number of pathogens such as bacteria, virus, fungus, parasites or prions. The most commonly known nosocomial pathogens include methicillin-resistant *Staphylococcus aureus* (MRSA), *Clostridium difficile* (*C. difficile*) and *Escherichia coli* (*E*. *coli*)*.*

*C. difficile* is a species of Gram-positive bacteria of the genus *Clostridium,* an anaerobic spore-forming bacillus. *C. difficile* is a commensal bacterium of the human intestine present in approximately 2-5% of the population. However, an imbalance of gut bacterial load and infection with *C. difficile* can result in severe diarrhoea and intestinal dysfunction. *C. difficile* infection can range in severity from asymptomatic to severe and life-threatening. Symptoms of the disease, such as diarrhoea, abdominal pain and fever, are usually caused by inflammation of the lining of the large intestine. In rare cases, *C. difficile* can lead to inflammation of the gut lining (Pseudomembranous colitis), blood poisoning (septicaemia) and tears in the large intestine (perforation of the colon).

*C. difficile* gut overpopulation and infection is particularly apparent in the elderly and also in patients where there is a reduction or eradication of commensal gut flora as a consequence of treatment with broad spectrum antibiotics. *C. difficile* is identified as the most common cause of antibiotic associated diarrhoea. Additionally, *C. difficile* infection is common in immune compromised or suppressed individuals, for example, those undergoing certain medical treatment. Similarly, some chronic diseases can present increased risk to infection (e.g. Diabetics have a general increased susceptibility to nosocomial infections). With the introduction of broad-spectrum antibiotics and chemotherapeutic antineoplastic drugs in the second half of the twentieth century, antibiotic and chemotherapy associated diarrhoea has become more common, with Pseudomembranous colitis first described as a complication of *C. difficile* infection in 1978.

People are most often infected with *C. difficile* in hospitals, nursing homes, or other medical institutions, although infection in the community is increasing. The rate of infection is estimated to be 13% in patients with hospital stays of up to 2 weeks, and 50% in those with hospital stays longer than 4 weeks. It has been estimated that in the UK in 2008 nearly 50,000 cases of *C. difficile* were recorded in patients aged 65 years or over along with a reported 6,000 deaths (*Office for national statistic, 2008: Health Statistics quarterly, 39*)*.* Currently, in the UK it costs approximately £400 per day to treat and manage infection in each patient. Given the estimated hospital duration of patients with *C. difficile* is between 14-27 days this, in addition to a serious health concern, represents a considerable, yet avoidable, cost burden (Cardiff & Vale NHS Trust, 2010).

The course of treatment of *C. difficile* infection varies according to its severity. For example, in mild to moderate infections caused as a consequence of antibiotic administration, ceasing the antibiotic treatment can re-establish the natural gut flora. In severe cases, *C. difficile* cytotoxic antibiotics, such as metronidazole or vancomycin, can be administered. However, antibiotic administration has to be carefully considered to avoid increasing the incidence of antibiotic resistant bacterial strains. Other treatment methods include re-establishment of the gut flora, for example, through use of probiotics such as *Saccharomyces boulardii* or *Lactobacillus acidophilus,* or by faecal bacteriotherapy (stool transplantation from an uninfected individual).

The pathogenesis of *C. difficile* is attributed to the production of toxins by certain strains. This includes the enterotoxin (*C. difficile* toxin A) and cytotoxin (*C. difficile* toxin B), which are both believed to be responsible for the diarrhoea and inflammation seen in infected patients. Toxins A and B are glucosyltransferases encoded by the tcdA and tcdB genes, respectively. They belong to the large clostridial cytotoxin (LCT) family, and share approximately 66% amino acid homology to each other. Consequently, the toxins have similar protein structure and target and inactivate the Rho-Rac family of GTPases in the host epithelial cells. This has been shown to result in actin depolymerisation by a mechanism correlated with a decrease in the ADP-ribosylation of the low molecular mass GTP-binding Rho proteins. This eventually results in massive fluid secretion, acute inflammation, and necrosis of the colonic mucosa. Different strains of *C. difficile* exist, which vary in the toxins that they secrete; it is believed that pathogenic strains produce both toxin A and toxin B whereas non toxigenic strains lack both toxins.

Consequently, there is a significant requirement to be able to detect *C. difficile* infection, with early diagnosis required to direct and simplify treatment, and also reduce the risk of bacterial spread. Several conventional methods are currently used to detect *C. difficile* infection although many of these methods are time-consuming and laborious, mainly attributed to a requirement for high sensitivity and specificity. The current diagnostic methods mainly consist of the detection of the *C. difficile* organisms or their toxins in faecal samples. Commonly used detection methods include toxinogenic assays, PCR methods, antigen detection (GDH) and ELISA based assays.

Cytotoxicity assays rely on toxigenic culture; this is a relatively sensitive and specific assay, in which stool samples are prepared appropriately and cultured on selective medium to test for *C. difficile* toxin production. The assay mainly detects the presence of TcdB as it is far more potent than TcdA in causing cytopathic changes in cultured cells. However, the technique is complex and has a slow turnaround time (24-72 h).

Assessment of the A and B toxins by enzyme-linked immunosorbent assay (ELISA) is also undertaken, utilising monoclonal antibodies to detect TcdA and/or TcdB. These techniques have a reported sensitivity of 63-99% and a specificity of 93-100%. However, despite being very specific, the technique is slow and labour-intensive. Furthermore, if there is a requirement for further testing this can be particularly time-consuming. Alternative stool tests have also been proposed, such as stool leukocyte measurements and stool lactoferrin levels, but these have limited diagnostic accuracy.

Other methods, such as real-time PCR, exist for detecting bacterial genes or toxins. However, these require sophisticated equipment and training, and are relatively time-consuming. Additionally, these techniques are often very expensive, and an inherent problem with many PCR-based methods is distinguishing between different strains of *C. difficile,* often failing to identify pathogenic versus non-pathogenic strains, and also the specific toxins they may express (toxinotype). At present, *C*. *difficile* PCR assays employ a DNA based detection system to amplify specific DNA targets within 2-8 hours. Typically, these assays are enzyme-based, and thus require prior purification to remove enzyme inhibitors which prevent the amplification of target DNA, which can result in false negative reactions. Additionally, poor DNA extraction methods and the presence of exogenous DNases and RNases within a reaction can lead to erroneous results. Therefore currently, it is very difficult to directly detect target *C*. *difficile* DNA from a faecal sample without prior purification when using PCR.

Furthermore all strains of *C*. *difficile* possess an intact toxin B encoding gene but show variations in the genes encoding toxin A. Consequently, PCR DNA based assays such as BD Gene Ohm and Xpert Cepheid are configured to detect only toxin B. However the role of toxins A and B in disease has been widely debated, with some suggesting only toxin B is essential for virulence and other suggesting both toxins are capable of causing fulminant disease. Furthermore, given Toxin A and toxin B share ∼66% sequence and functional homology, and are thought to have evolved from a gene duplication event, many methods are unable to distinguish between different toxinotypes. This highlights the importance of a diagnostic system able to detect both toxins A and B in an individual, with current methods failing to provide a quick and reliable method that can distinguish between different strains and toxinotypes.

The growing incidence and severity of *C. difficile* infections indicates a need to develop a rapid detection method for *C*. *difficile* to prevent transfer of micro-organisms between patients, and to enable healthcare professionals to provide an efficient treatment regimen for the patient. Further, the prevention of cross infection between patients would reduce treatment times and the cost burden on healthcare systems. Current detection methods are time-consuming, and often lack sensitivity. Symptoms of *C. difficile* infection normally manifest within 24-48 hours of infection, and consequently there is a pressing need to diagnose *C*. *difficile* during the early, subclinical phase of infection to enable treatment and minimise cross transmission within the hospital environment. A bioassay capable of detecting the presence of *C. difficile* in clinical samples in <60 seconds without the need for complex preprocessing would dramatically reduce the time required to obtain confirmation of the presence of the organism compared to current laboratory assays. Ideally such an assay would be able to detect *C*. *difficile* with high sensitivity and specificity (i.e. be capable of accurately detecting all variant strains and toxinotypes of the organism), whilst being inexpensive to run and low maintenance.

The genes encoding toxin A and B are thought to have evolved as a result of horizontal gene transfer. It is generally thought that gene sequences encoding biologically essential protein structures for any organism are under considerable selective pressure, and therefore are conserved. Indeed, within the genome of *C*. *difficile* areas of sequence conservation have been identified (Rupnik *et a*/*.,* 1998). In our investigations, we have identified regions within the *C. difficile* genome that serve as target regions for identifying the organism. Having identified these regions we have designed and synthesised oligonucleotide probes specific to these target regions.

Through a rigorous screening assay we have tested the identified oligonucleotide probes against a comprehensive panel of 58 *C*. *difficile* clinical isolates, representing a range of differing toxinotypes. These probes were further screened for specificity against isolates from near neighbours within the *Clostridium* family, including those which possessed LCT genes. Finally they were subjected to a more stringent analysis by running them against 10 metagenomic DNA extracts from human gut flora which represents approximately 10¹² per gram wet weight bacteria.

We have unexpectedly found that targeting these specific regions provides highly specific and sensitive detection of *C. difficile* genes, which thus can be used to accurately detect the presence of *C. difficile* in a sample. Therefore, through selective screening we have identified target regions of toxin A and B of *C*. *difficile,* which can be detected, using hybridization, to enable the production of a reliable and repeatable screening and diagnostic assay for the detection of *C. difficile* in a sample. Furthermore, these regions have been shown to be highly *C. difficile* specific permitting identification of a variety *C. difficile* strains and toxinotypes, and so are suitable for inclusion in an assay able to detect the presence of pathogens in a rapid screening method.

To exemplify the specificity and sensitivity of these identified target regions, in one embodiment of the invention, we used a Microwave accelerated metal enhanced fluorescence (MAMEF) platform, which has previously been used to detect a range of bacterial pathogens including *B. anthracis, Salmonella typhimurium* and *C*. *trachomatis* (Asian et al., 2008; Zhang et al., 2011, Tennant et al., 2011). This technology breaks open vegetative bacteria and spores, releasing target DNA which is rapidly detected by pathogen specific probes. We have been able to show that by binding probes to our conserved target regions in a rapid MAMEF based assay, we are able to detect as few as 100 spores in a 500 µl faecal suspension within 40 seconds. To put this into context, this represents a high level of sensitivity as in human infection approximately 10⁶- 10⁸ spores are released. This level of sensitivity and speed is superior compared to current *C*. *difficile* detection methods. Indeed, there is presently no assay capable of directly detecting the presence of *C. difficile* within faecal samples. Therefore the detection of the identified target regions can be developed into a highly specific and accurate real-time diagnostic and screening assay able to detect both toxin A and B encoding genes of *C. difficile.* This has important implications in the screening of patients upon admission to hospital to enable appropriate treatment regimens and clinical management decisions to be made.

### Statements of Invention

According to a first aspect of the invention, there is provided a method for the detection of *C*. *difficile* in a sample wherein said *C. difficile* comprises the following nucleic acid target region characterised by the following sequence:
I) Tcaagactctattatagtaagtgcaaatcaatatgaagt;
which method comprises exposing said sample to:
a) at least one oligonucleotide probe that is complementary to at least a part of said target region, or;
b) at least one oligonucleotide probe that is complementary to at least a part of a degenerate version of said target region or;
c) an oligonucleotide probe that is at least 80% homologous to the oligonucleotides in a) or b); and
detecting the binding of said oligonucleotide to said target region and, where binding occurs, concluding that *C. difficile* is present in said sample.

In a preferred embodiment of the invention, the target region relates to *C. difficile* toxin B and is used in the method for the detection of *C. difficile* in a sample. As will be appreciated by those skilled in the art, Toxin A is absent, or truncated in some variant strains of *C. difficile* whilst toxin B is present in all strains. Consequently, testing for the target region in Toxin B will provide the most accurate method for the detection of *C. difficile* in a sample.

However, in a preferred embodiment of the invention a further test may be undertaken on said sample, sequentially or simultaneously, with the above method to determine if the following further nucleic acid target region having the following sequence is present:
II) Aaaatattactttaatactaacactgctgttgcagttactggatggcaaactattaatggtaaaaaatact acttt;
which method comprises exposing said sample to:
d) at least one oligonucleotide probe that is complementary to at least a part of said further nucleic acid target region, or;
e) at least one oligonucleotide probe that is complementary to at least a part of a degenerate version of said further nucleic acid target region or;
f) an oligonucleotide probe that is at least 80% homologous to the oligonucleotides in d) or e); and
detecting the binding of said oligonucleotide to said further nucleic acid target region and, where binding occurs, concluding that *C*. *difficile* is present in said sample.

In this embodiment of the invention said further nucleic acid target region relates to *C. difficile* toxin A.

In a preferred aspect of the invention said sample is any matter suspected of containing *C. difficile,* such as but not limited to, earth, soil, a gut sample, faeces, urine, body fluid, food, laboratory cultures, hospital equipment, or wound dressings.

Most ideally said sample is taken from the gut, such as a sample of faeces and said method is a diagnostic method for identifying individuals, typically patients, carrying said infection. Ideally, said sample is treated to extract DNA therefrom, typically, by using conventional techniques as described herein and as known to those skilled in the art.

In a further preferred aspect of the invention said oligonucleotide is designed for use in a conventional oligonucleotide binding assay, such as but not limited to, PCR, Southern Blotting, DNA dot blotting, DNA microarray techniques, Microwave-assisted annealing (hybridisation) assays including metal enhanced fluorescence.

As will be appreciated by those skilled in the art, in the instance where said oligonucleotide is to be used in a PCR reaction it, advantageously, is designed to have optimal properties for performing such a technique, such as but not limited to, a GC content of approximately 50%, minimal self-complementarity, an optimal nucleotide length of between 15 and 50 nucleotide base pairs, or the like.

In a yet a further preferred embodiment of the invention, said oligonucleotide comprises a signaling molecule which emits a signal when said oligonucleotide exists in either a bound or an unbound state; or, alternatively, it emits a quantitative signal whose scale is representative of at least one of said states. Ideally, said signaling molecule is located immediately next to the binding nucleotides of said oligonucleotide probe. Alternatively, said signaling molecule is located distally from the binding nucleotides of said oligonucleotide probe due to the presence of at least one nucleotide base, or preferably 5, 10, 11, 12, 13, 14 or 15 nucleotide bases, that does not bind said target region. As will be appreciated by those skilled in the art, this may improve accessibility of the signaling molecule for detection whilst also reducing the possibility of the signaling molecule interfering with binding of the oligonucleotide probe to the target site.

Additionally or alternatively, said oligonucleotide comprises a part of a signaling system which part interacts with other parts of said system to emit a signal when either in a bound state or an unbound state whereby the binding of said oligonucleotide to said target site can be detected. As will be appreciated by those skilled in the art, there are numerous examples of such systems such as, but not limited to, conjugation of said oligonucleotide to Alkaline Phosphatase or Horseradish Peroxidase, which catalyse the cleavage of substrates to generate a signal.

In the above embodiments of the invention said signaling molecule may be a fluorescent molecule or a chemiluminescent molecule or a bioluminescent molecule or, indeed, any molecule that provides a detectable signal when said oligonucleotide exists in either a bound or unbound state. As will be appreciated by those skilled in the art, this may include but is not limited to FAM (5-or 6-carboxyfluorescein), VIC, NED, Fluorescein, Digoxigenin, FITC, IRD- 700/800, CY3, CY5, CY3.5, CY5.5, Cy7, HEX, TET, TAMRA, JOE, ROX, BODIPY TMR, Oregon Green, Rhodamine Green, Hydroxycumarin, Aminocoumarin, Lucifer Yellow, TruRed, Rhodamine Red, Texas Red, Yakima Yellow, Alexa Fluor, PET Biosearch Blue(TM), Marina Blue(R), Bothell Blue(R), CAL Fluor(R) Gold, CAL Fluor(R) Red 610, Quasar(TM) 670, LightCycler Red640(R), Quasar(TM) 705, LightCycler Red705(R), or the like.

In alternative embodiments of the invention said oligonucleotide probe comprises a pair of oligonucleotide probes, one of which is an anchor probe that binds target DNA, further, another of which is a labelled detector probe that binds at a distance, with respect to said anchor probe, and so positions the label, such as a fluorophore, optimally for biomolecular recognition to occur. Advantageously, the use of anchor and detector probes also allows for two levels of sensitivity within the assay for each toxin as two distinct regions of the same target are bound thus reducing the probability that the signal relates to specific binding or increasing the probability that the signal represents specific binding, rather than background noise.

Ideally, said anchor probe binds a site separated by 1-50 nucleotides from the site bound by the detector probe, or vice versa. More ideally still said anchor probe binds a site separated by 3-20 nucleotides from the site bound by the detector probe, or vice versa. Most ideally, said anchor probe binds a site separated by 5 nucleotides from the site bound by the detector probe, or vice versa. Those skilled in the art will appreciate similar modifications can be made to these ideal structures providing binding and/or signaling functionality is retained.

In yet a further preferred embodiment of the invention, said anchor probe further comprises a chemical group/molecule that can attach, or adhere, to a selected surface. Ideally, said chemical group/molecule is located adjacent to, or distal from, the binding nucleotides, as opposed to in the binding region of the oligonucleotide. A suitable chemical group/molecule includes, but is not limited to, a thiol group or biotin. As will be appreciated by those skilled in the art, this permits binding of the anchor probe to an immobilized substrate, for example, for production of array platforms.

In yet a further preferred embodiment of the invention, said anchor probe further comprises at least one T nucleotide base, or most ideally between 1 and 10 T nucleotide bases between said chemical group/molecule and the binding region. As will be appreciated by those skilled in the art, such modifications increase the flexibility of the anchor probe on immobilized substrates to increase maximal binding of target sequences. Those skilled in the art will appreciate similar modifications can be made to these ideal structures to achieve the same effect providing binding and/or signaling functionality is retained.

In a further preferred embodiment, a plurality of oligonucleotide probes may be used to detect the presence or absence of said target regions in the same sample. Moreover, a plurality of different oligonucleotide probes, each labelled with the same or different signalling molecules, may be used to detect the presence or absence of said target regions in the same sample.

In yet a further preferred embodiment of the invention, the concentration of said oligonucleotide probes is typically and without limitation between 10-100 ng/ul and ideally between 40-80ng/ul, most ideally between 55-65ng/ul including all 1ng/ul integers there between. In this way, the oligonucleotide probes are at a concentration to permit sensitive and accurate detection of said target sequences. However, as will be appreciated by those skilled in the art, the concentration of oligonucleotide probes will vary according to the particular oligonucleotide binding assay utilised to permit optimal binding and target detection.

In part c) above the skilled person will appreciate that homologues or derivatives of the oligonucleotides of the invention will also find use in the context of the present invention. Thus, for instance oligonucleotides which include one or more additions, deletions, substitutions or the like are encompassed by the present invention. A software program such as BLASTx can be used to identify oligonucleotide probes with the requisite at least 80% homology. This program will align the longest stretch of similar sequences and assign a homology value to the fit. It is thus possible to obtain a comparison where several regions of similarity are found, each having a different score. This type of analysis is contemplated in the present invention.

The term "homologous" as used herein refers to oligonucleotide sequences which have a sequence at least 80% homologous (or identical) to the said oligonucleotide sequence of the probes in parts a) or b). It is preferred that homologues are at least 81 % homologous to the probes in parts a) or b) and, in increasing order of preference, at least 82%, 83%, 84%, 85%, 86%, 87%, 88%, 89%, 90%, 91%, 92%, 93%, 94%, 95%, 96%, 97%, 98% or 99% homologous to the probes in parts a) or b).

Most ideally, said probes are between 15 - 30 nucleotides in length and most ideally 15 - 25 nucleotides in length and even more ideally 17 - 22 nucleotides in length.

Most ideally still, said oligonucleotides are selected from the group comprising:
Toxin A tactttaatactaacac; tttaatactaacactgc; actaacactgctgttgc; tgctgttgcagttactg; tgctgttgcagttactggatgg; tgttgcagttactggatggcaa; atggcaaactattaatggtaaa; gatggcaaactattaatggtaa;
Toxin B tcaagactctattatag; or taagtgcaaatcaatatgaagt.

Yet more ideally still, said oligonucleotides are selected from the group comprising:
Toxin A tttaatactaacactgc; tgttgcagttactggatggcaa;
Toxin B tcaagactctattatag; or taagtgcaaatcaatatgaagt.

Yet more ideally still, said oligonucleotides are selected from the group comprising:
Toxin B ttaatgccatctataag; aatgccatctataagtc; gccatctataagtcaag; catctataagtcaagac; tctataagtcaagactc; ctataagtcaatatgaagttag; aatatgaagttagaataaatag; tatgaagttagaataaatagtg; aagttagaataaatagtgaagg.

Yet more ideally still, said oligonucleotides are selected from the group comprising:
Toxin A ctgcgaactattgatgg; atggtaaaaaatattac; aaatattactttaatac; attactttaatactaac; tactttaatactaacac; gtaaaaaatattactttaatac; aaaatattactttaatactaac; aatattactttaatactaacac; attactttaatactaacactgc; tactttaatactaacactgctg; ttaatactaacactgctgttgc; and aatactaacactgctgttgcag.

In use, it will therefore be appreciated that the invention involves the identification of at least one target region or nucleic acid sequence in species of *Clostridium* bacteria, which can be used to accurately and sensitively determine the presence or absence of said bacteria in a sample. As will be appreciated by those skilled in the art, the detection of the said target region or nucleic acid sequence can be achieved by numerous techniques, including without limitation, oligonucleotide binding assays. The present invention therefore also discloses complementary oligonucleotide binding probes for said target region or nucleic acid sequence, or probes with a degree of sequence similarity thereto, for use in assays for the detection of the disclosed target region of the invention. Additionally, as is known to those skilled in the art, transcripts of the said target region or nucleic acid sequence can also be detected in the working of the invention, such as but not limited to, the detection of mRNA encoded by said region or sequence in the technique of RT-PCR.

According to a second aspect of the invention, there is provided a kit for the detection of *C. difficile* in a sample wherein said *C*. *difficile* comprises the following nucleic acid target region characterised by the following sequence:
I) tcaagactctattatagtaagtgcaaatcaatatgaagt;
which kit comprises:
a) at least one oligonucleotide probe that is complementary to at least a part of said target region, or;
b) at least one oligonucleotide probe that is complementary to at least a part of a degenerate version of said target region, or;
c) an oligonucleotide probe that is at least 80% homologous to oligonucleotides a) or b) and
optionally, reagents and/or instructions for practicing said detection.

In a preferred kit of the invention a further test may be undertaken on said sample, sequentially or simultaneously, with the above method to determine if the following further nucleic acid target region having the following sequence is present:
II) Aaaatattactttaatactaacactgctgttgcagttactggatggcaaactattaatggtaaaaaatact acttt;
and which kit further comprises:
d) at least one oligonucleotide probe that is complementary to at least a part of said further nucleic acid target region, or;
e) at least one oligonucleotide probe that is complementary to at least a part of a degenerate version of said further nucleic acid target region or;
f) an oligonucleotide probe that is at least 80% homologous to the oligonucleotides in d) or e); and
detecting the binding of said oligonucleotide to said further nucleic acid target region and, where binding occurs, concluding that *C*. *difficile* is present in said sample.

In a further preferred embodiment of the second aspect of the invention said oligonucleotide is designed for use in a conventional oligonucleotide binding assay, such as but not limited to, PCR, Southern Blotting, DNA dot blotting, DNA microarray techniques, Microwave-assisted annealing (hybridisation) assays including metal enhanced fluorescence.

In a yet a further preferred embodiment of the second aspect of the invention, said oligonucleotide comprises a signaling molecule which emits a signal when said oligonucleotide exists in either a bound or an unbound state; or it emits a quantitative signal whose scale is representative of at least one of said states.

Additionally or alternatively, said oligonucleotide comprises a part of a signaling system which part interacts with other parts of said system to emit a signal when either in a bound state or an unbound state whereby the binding of said oligonucleotide to said target site can be detected. As will be appreciated by those skilled in the art, there are numerous examples of such systems such as, but not limited to, conjugation of said oligonucleotide to Alkaline Phosphatase or Horseradish Peroxidase, which catalyse the cleavage of substrates to generate a signal.

In alternative embodiments of the invention said oligonucleotide probe comprises a pair of oligonucleotide probes, one of which is an anchor probe that binds target DNA, further, another of which is a labelled detector probe that binds at a distance, with respect to said anchor probe, and so positions the label, such as a fluorophore, optimally for biomolecular recognition to occur. Advantageously, the use of anchor and detector probes also allows for two levels of sensitivity within the assay for each toxin. Ideally, said anchor probe binds a site separated by 1-50 nucleotides from the site bound by the detector probe, or vice versa. More ideally still said anchor probe binds a site separated by 3-20 nucleotides from the site bound by the detector probe, or vice versa. Most ideally, said anchor probe binds a site separated by 5 nucleotides from the site bound by the detector probe, or vice versa. Those skilled in the art will appreciate similar modifications can be made to these ideal structures providing binding and/or signaling functionality is retained.

In yet a further preferred embodiment of the invention, said anchor probe further comprises a chemical group/molecule that can attach, or adhere, to a selected surface. Ideally, said chemical group/molecule is located adjacent to, or distal from, the binding nucleotides, as opposed to in the binding region of the oligonucleotide. A suitable chemical group/molecule includes, but is not limited to, a thiol group or biotin. As will be appreciated by those skilled in the art, this permits binding of the anchor probe to an immobilized substrate, for example, for production of array platforms.

In yet a further preferred embodiment of the invention, said anchor probe further comprises at least one T nucleotide base, or most ideally between 1 and 10 T nucleotide bases between said chemical group/molecule and the binding region. As will be appreciated by those skilled in the art, such modifications increase the flexibility of the anchor probe on immobilized substrates to increase maximal binding of target sequences. Those skilled in the art will appreciate similar modifications can be made to these ideal structures to achieve the same effect providing binding and/or signaling functionality is retained.

In a further preferred embodiment, a plurality of oligonucleotide probes may be used to detect the presence or absence of said target regions in the same sample. Moreover, a plurality of different oligonucleotide probes, each labelled with the same or different signalling molecules, may be used to detect the presence or absence of said target regions in the same sample.

In yet a further preferred embodiment of the invention, the concentration of said oligonucleotide probes is typically and without limitation between 10-100 ng/ul and ideally between 40-80ng/ul, most ideally between 55-65ng/ul including all 1ng/ul integers there between. In this way, the oligonucleotide probes are at a concentration to permit sensitive and accurate detection of said target sequences. However, as will be appreciated by those skilled in the art, the concentration of oligonucleotide probes will vary according to the particular oligonucleotide binding assay utilised to permit optimal binding and target detection.

In part c) above the skilled person will appreciate that homologues or derivatives of the oligonucleotides of the invention will also find use in the context of the present invention. Thus, for instance oligonucleotides which include one or more additions, deletions, substitutions or the like are encompassed by the present invention. A software program such as BLASTx can be used to identify oligonucleotide probes with the requisite at least 80% homology. This program will align the longest stretch of similar sequences and assign a homology value to the fit. It is thus possible to obtain a comparison where several regions of similarity are found, each having a different score. This type of analysis is contemplated in the present invention.

The term "homologous" as used herein refers to oligonucleotide sequences which have a sequence at least 80% homologous (or identical) to the said oligonucleotide sequence of the probes in parts a) or b). It is preferred that homologues are at least 81%, homologous to the probes in parts a) or b) and, in increasing order of preference, at least 82%, 83%, 84%, 85%, 86%, 87%, 88%, 89%, 90%, 91%, 92%, 93%, 94%, 95%, 96%, 97%, 98% or 99% homologous to the probes in parts a) or b).

Most ideally, said probes are between 15 - 30 nucleotides in length and most ideally 15 - 25 nucleotides in length and even more ideally 17 - 22 nucleotides in length.

Most ideally still, said oligonucleotides are selected from the group comprising:
Toxin A tactttaatactaacac; tttaatactaacactgc; actaacactgctgttgc; tgctgttgcagttactg; tgctgttgcagttactggatgg; tgttgcagttactggatggcaa; atggcaaactattaatggtaaa; gatggcaaactattaatggtaa;
Toxin B tcaagactctattatag; or taagtgcaaatcaatatgaagt.

Yet more ideally still, said oligonucleotides are selected from the group comprising: Toxin A tttaatactaacactgc; tgttgcagttactggatggcaa;
Toxin B tcaagactctattatag; or taagtgcaaatcaatatgaagt.

Yet more ideally still, said oligonucleotides are selected from the group comprising:

Yet more ideally still, said oligonucleotides are selected from the group comprising:

In yet a further aspect of the invention there is provided an array comprising any one or more of the above oligonucleotides probes, including any combination thereof and, ideally, all of said oligonucleotides probes.

In yet a further aspect of the invention there is provided an oligonucleotide probe as described herein.

In the claims which follow and in the preceding description of the invention, except where the context requires otherwise due to express language or necessary implication, the word "comprises", or variations such as "comprises" or "comprising" is used in an inclusive sense i.e. to specify the presence of the stated features but not to preclude the presence or addition of further features in various embodiments of the invention.

All references, including any patent or patent application, cited in this specification are hereby incorporated by reference. No admission is made that any reference constitutes prior art. Further, no admission is made that any of the prior art constitutes part of the common general knowledge in the art.

Preferred features of each aspect of the invention may be as described in connection with any of the other aspects.

Other features of the present invention will become apparent from the following examples. Generally speaking, the invention extends to any novel one, or any novel combination, of the features disclosed in this specification (including the accompanying claims and drawings). Thus, features, integers, characteristics, compounds or chemical moieties described in conjunction with a particular aspect, embodiment or example of the invention are to be understood to be applicable to any other aspect, embodiment or example described herein, unless incompatible therewith.

Moreover, unless stated otherwise, any feature disclosed herein may be replaced by an alternative feature serving the same or a similar purpose.

The present invention will now be described by way of example only with particular reference to the following figures wherein:

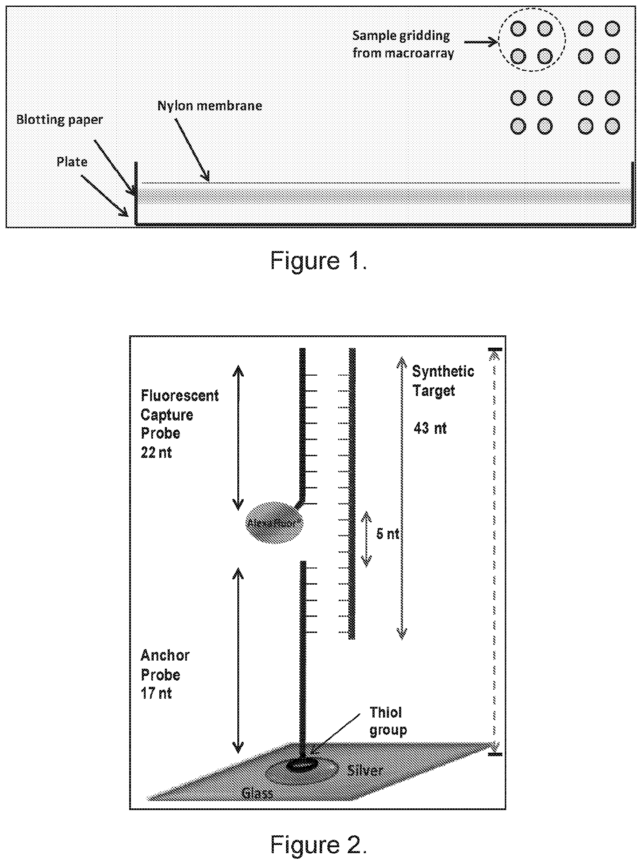

**Figure 1****.** A schematic view of the DNA dot blot forming the macroarray. Genomic DNA from different *C*. *difficile* isolates was macroarrayed onto a positively charged nylon membrane in four replicate dots. gDNA was post-fixed using UV light. This was performed for all probes from both target regions (tcdA76bp and tcdB39bp);
**Figure 2****.** A schematic view of the oligonucleotide probe design of the invention as utilised in the MAMEF assay The anchor probe is fixed to the SiF by the addition of the thiol group and is 17 oligonucleotides in length. The detector probe is 22 oligonucleotides in length and has a 3' Alexa® fluor moiety. Binding of the target to the anchor DNA and detector DNA causes release of plasmons, which fluoresce under laser light. The five nucleotide spacer region is included to aid the fluorescent reaction to occur;

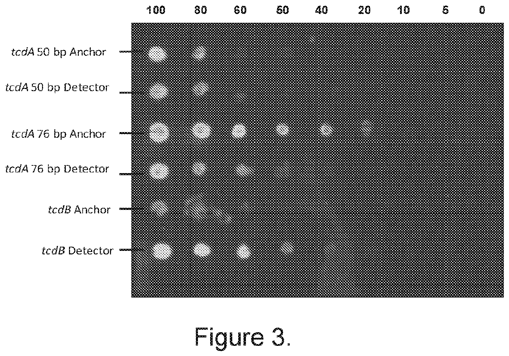

**Figure 3****.** Dot-blot analysis to determine the optimum sensitivity of DIG-labelled oligonucleotide probes targeting conserved regions of toxin A and B of *C. difficile.* Probes tested in this dot blot analysis were from both target regions (tcdA76bp and tcdB39bp);

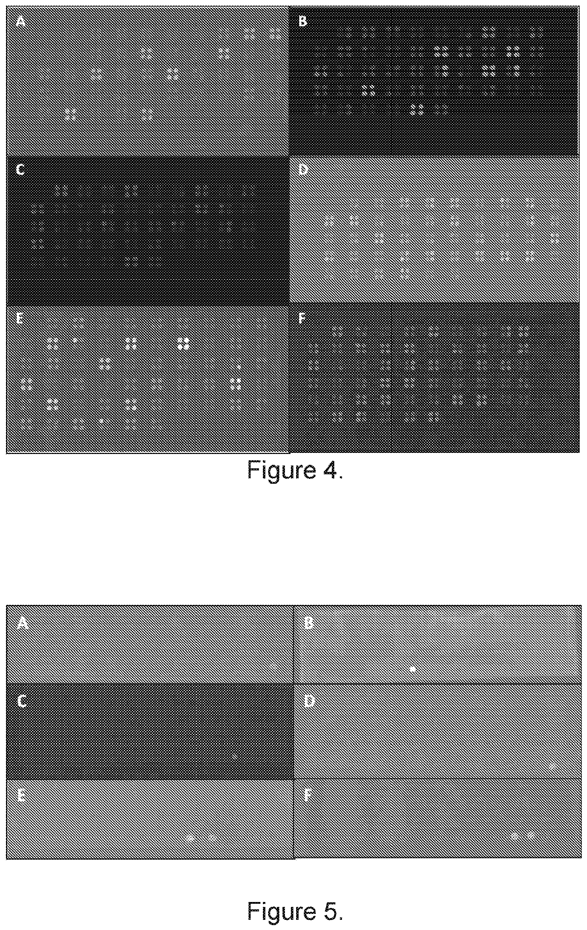

**Figure 4****.** Macroarray analysis of gDNA from the *C. difficile* isolates using oligonucleotide probes targeting regions of toxin A and B, arranged according to the PCR ribotype as shown in table 8. [A] *tcdA50* anchor probe, [B] *tcdA50* detector probe, [C] *tcdA76* anchor probe, [D] *tcdA76* detector probe, [E] *tcdB* anchor probe [F] *tcdB* detector probe. Probes tested in this dot blot analysis were from both target regions (tcdA76b and tcdB39bp);
**Figure 5****.** Macroarray analysis of gDNA from unrelated bacterial species using oligonucleotide probes targeting regions of toxin A and B. Positive control for all reactions is CD630 and a variant strain control R22680 (*tedA*⁻*tcdB*⁺) was also included (highlighted by white square). [A] *tcdA50* anchor probe, [B] *tcdA50* detector probe, [C] *tcdA76* anchor probe, [D] *tcdA76* detector probe, [E] *tcdB* anchor probe, [F] *tcdB* detector probe. Probes tested in this dot blot analysis were from both target regions (tcdA76bp and tcdB39bp);

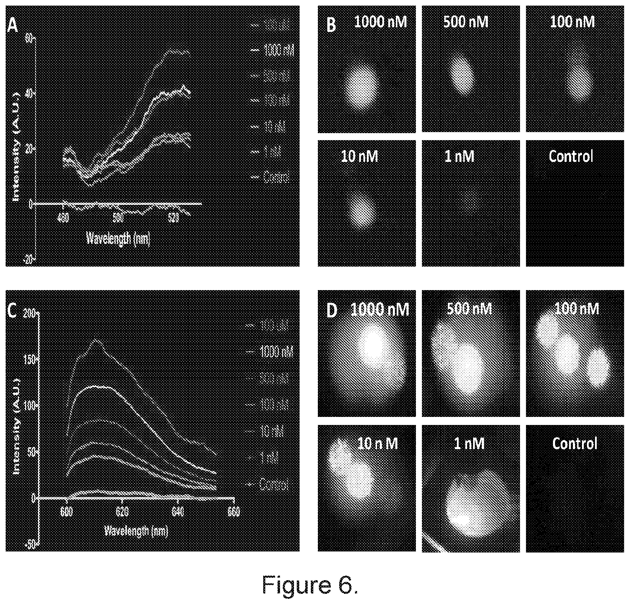

**Figure 6****.** Detection of various concentrations of oligonucleotide probes targeting conserved regions of *C. difficile* toxin A and B in TE buffer by MAMEF. [A] Toxin A oligonucleotide probe. [B] The fluorescent signal produced at each concentration of toxin A oligonucleotide probe. [C] Toxin B oligonucleotide probe. [D] The fluorescent signal produced at each concentration of toxin B oligonucleotide probe. Probes tested in this MAMEF assay were from both target regions (tcdA76bp and tcdB39bp);

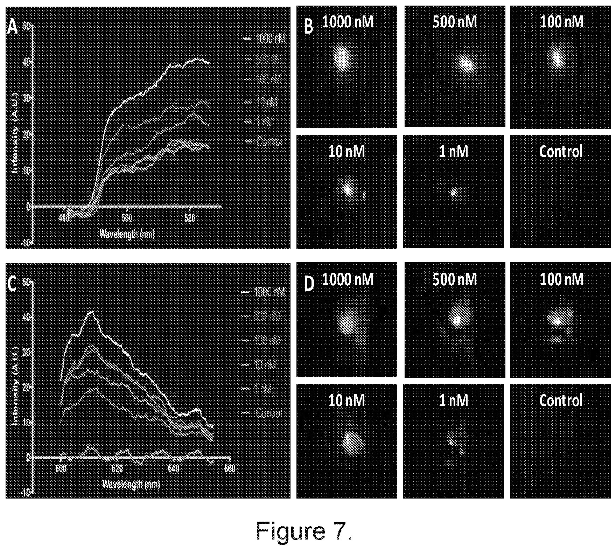

**Figure 7****.** Detection of various concentrations of oligonucleotide probes targeting conserved regions of *C*. *difficile* toxin A and B in whole milk by MAMEF. [A] Toxin A oligonucleotide probe. [B] The fluorescent signal produced at each concentration of toxin A oligonucleotide probe. [C] Toxin B oligonucleotide probe. [D] The fluorescent signal produced at each concentration of toxin B oligonucleotide probe. Probes tested in this MAMEF assay were from both target regions (tcdA76bp and tcdB39bp);

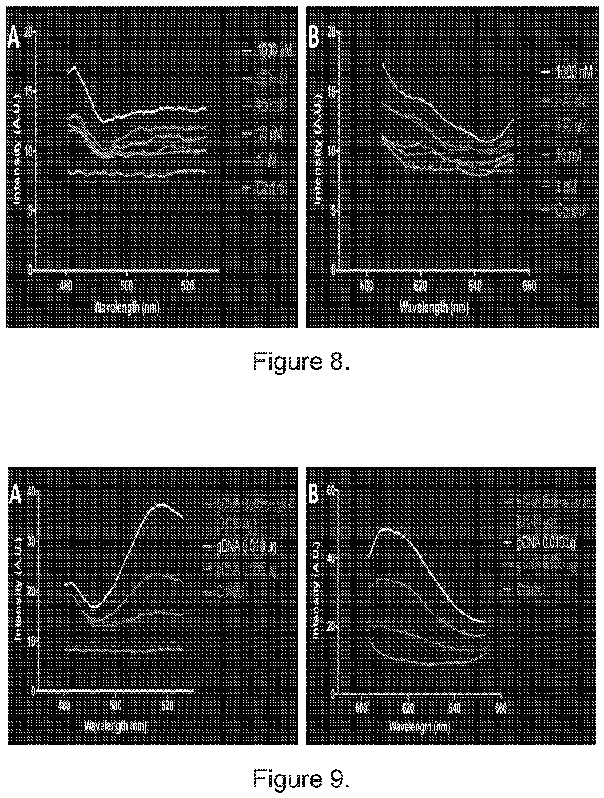

**Figure 8****.** Detection of various concentrations of oligonucleotide probes targeting conserved regions of *C. difficile* toxin A and B in faecal matter by MAMEF. [A] Toxin A oligonucleotide probe [B] Toxin B oligonucleotide probe. Probes tested in this MAMEF assay were from both target regions (tcdA76bp andtcdB39bp);
**Figure 9****.** Detection of oligonucleotide probes targeting conserved regions of *C. difficile* toxin A and B within various concentrations of genomic DNA in human faecal matter by MAMEF. [A] Toxin A oligonucleotide probe. [B] Toxin B oligonucleotide probe. Probes tested specifically in this MAMEF assay were from both target regions (tcdA76bp and tcdB39bp);

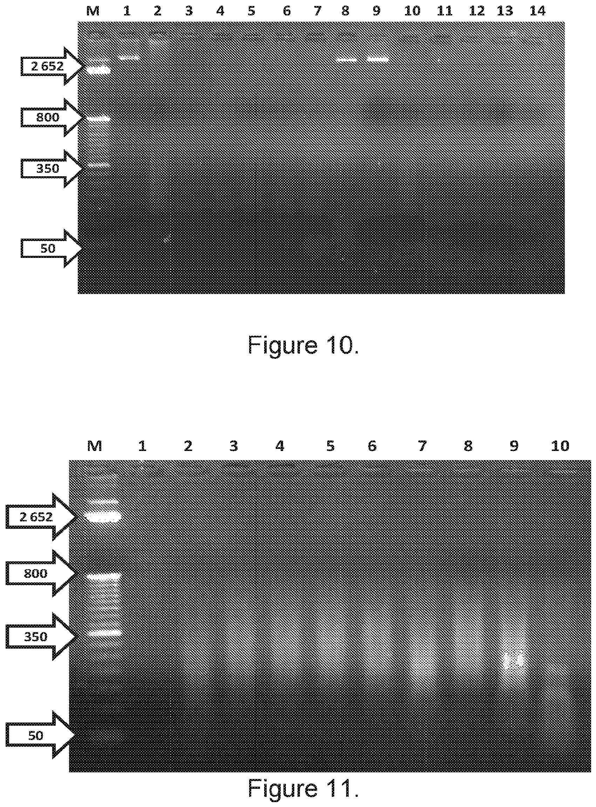

**Figure 10****.** Gel electrophoresis of boiled *C. difficile* spores and vegetative cell mixed spore preparation at a concentration of 1 x 105 cfu/ml were boiled in water and PBS buffer to ascertain DNA release. A sample was taken every half an hour for 3 h. Lanes 1-7: Boiling in PBS: Lane 1: shows the control sample of spores without boiling or centrifugation, Lane 2: Spore DNA release after 30 min boiling, Lane 3: Spore DNA release after 60 min boiling, Lane 4: Spore DNA release after 90 min boiling, Lane 5: Spore DNA release after 120 min boiling, Lane 6: Spore DNA release after 1500 min boiling, Lane 7: S Spore DNA release after 180 min boiling; Lanes 5-14: Boiling in Water: Lane 8: shows the control sample of spores without boiling or centrifugation, Lane 9: Spore DNA release after 30 min boiling, Lane 10: Spore DNA release after 60 min boiling, Lane 11: Spore DNA release after 90 min boiling; Lane 12: Spore DNA release after 120 min boiling; Lane 13: Spore DNA release after 150 min boiling, Lane 14: Spore DNA release after 180 min boiling;
**Figure 11****.** Gel electrophoresis of *C*. *difficile* spores and vegetative cell mixed preparations at a concentration of 1 x 105 cfu/ml lysed using the gold lysing triangles. The power used was 80% microwave power. The 1 kb marker (M) was used. Lane 1 represents the control which was not lysed. Lane 2: lysis at 8 s, Lane 3: 9 s, Lane 4: 10 s, Lane 5: 11 s, Lane 6: 12 s, Lane 7: 13 s, Lane 8: 14 s, Lane 9: 15 s and Lane 10: 16 s;

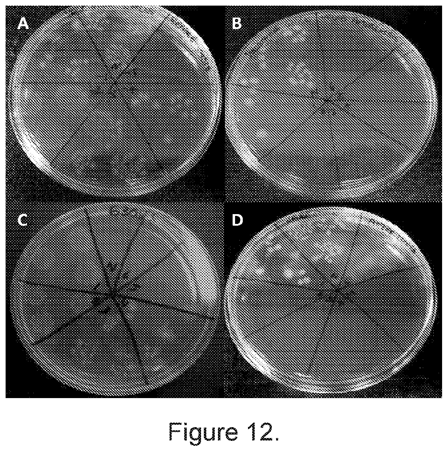

**Figure 12****.** Viable counts of *C. difficile* spores and vegetative cells before and after lysis in PBS. [A] *C. difficile* vegetative cells were lysed by microwave irradiation at a concentration of 4 x 10⁶ cfu/ml. [B] After lysis the concentration of vegetative cells was 6.67 x 10² cfu/ml. [C] Spores were lysed at a concentration of 1.33 x 10⁷ cfu/ml. [D] After lysis 3.67 x 10³ were detected;

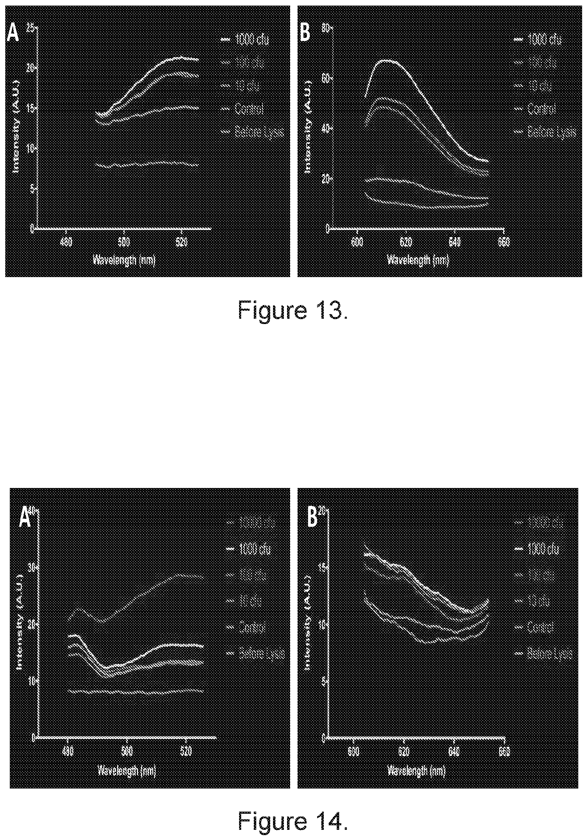

**Figure 13****.** Detection of oligonucleotide probes targeting regions of *C. difficile* toxin A and B from microwaved vegetative *C. difficile* cells in PBS using MAMEF. [A] Toxin A oligonucleotide probe. [B] Toxin B oligonucleotide probe. Probes tested in this MAMEF assay were from both target regions (tcdA76bp and tcdB39bp);
**Figure 14****.** Detection of oligonucleotide probes targeting regions of *C*. *difficile* toxin A and B from microwaved *C. difficile* spore preparation in PBS using MAMEF. [A] Toxin A oligonucleotide probe. [B] Toxin B oligonucleotide probe Probes tested in this MAMEF assay were from both target regions (tcdA76bp and tcdB39bp);

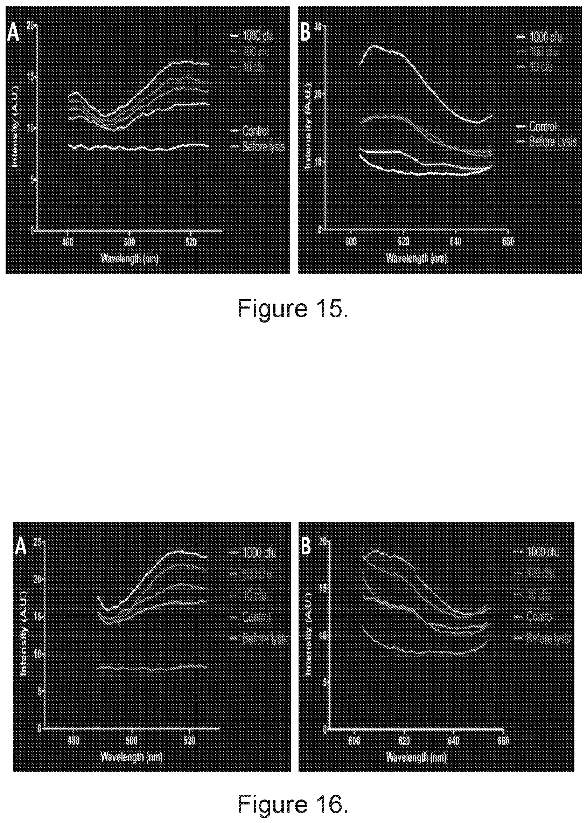

**Figure 15****.** Detection of oligonucleotide probes targeting regions of *C*. *difficile* toxin A and B from microwaved *C. difficile* spore preparation in milk using MAMEF. [A] Toxin A oligonucleotide probe. [B] Toxin B oligonucleotide probe. Probes tested in this MAMEF assay were from both target regions (tcdA76bp and tcdB39bp);
**Figure 16****.** Detection of oligonucleotide probes targeting regions of *C*. *difficile* toxin A and B from microwaved *C*. *difficile* spore preparation in human faecal matter using MAMEF. [A] Toxin A oligonucleotide probe. [B] Toxin B oligonucleotide probe. Probes tested in this MAMEF assay were from both target regions (tcdA76bp and tcdB39bp);

**Table 1.** The additional isolates of *C*. *difficile* are listed. Isolates from blood culture are listed as (B/C). Toxin production for each strain and its PCR ribotype are shown, with additional information including the source;
**Table 2.** Additional bacterial species used in this study. The species related to *C*. *difficile* are identified;
**Table 3.** Table of oligonucleotide probes targeting regions of toxin A and toxin B of *C. difficile* for use in MAMEF. The anchor probes for both toxins have a run of 5 consecutive Ts (thymine bases) included to increase the flexibility of the probes in detecting target DNA once bound to the silver surface. The capture probes have an Alexa ® group added to aid fluorescent detection. The target region negative strand was also synthesised to aid assay development. Probes tested in this MAMEF assay were from both target regions (tcdA76bp and tcdB39bp);
**Table 4.** Target regions of *C*. *difficile* toxin A and B identified by Multiple Sequence Alignment. Regions of toxin A and toxin B of *C*. *difficile* were identified by ClustalX and the MSA analysis software JalviewTM. Two regions in toxin A and three regions in toxin B were conserved across all the sequences collated from GenBank. Target probes were designed from both target regions: (tcdA76bp and tcdB39bp);
**Table 5.** Design of oligonucleotide probes targeting regions of toxin A of *C. difficile* identified in table 4. The entire conserved region and the anchor and detector probes designed from them is shown. Colour coding indicates the region targeted by the anchor probe and its corresponding detector probe. Target probes were designed from target region: (tcdA76bp);
**Table 6.** Design of oligonucleotide probes targeting regions of toxin B of *C*. *difficile* identified in table 4. The entire conserved region and the anchor and detector probes designed from them is shown. Colour coding indicates the region targeted by the anchor probe and its corresponding detector probe. Whilst the length of the probes are indicated as 17nt or 22nt there is some slight variation in this length for two of the probes;
**Table 7.** Oligonucleotide probes targeting regions of toxin A and B of *C. difficile* used in further dot-blot analysis and MAMEF assays. Probes were from both target regions (tcdA76bp, tcdB39bp);
**Table 8.** Genomic DNA from the collection of *C. difficile* isolates tested was arrayed onto the positively charged membrane in the format shown. Isolates were organised according to PCR ribotype. This assay was performed for all probes from both target regions (tcdA76bp and tcdB39bp); and
**Tables 9 & 10.** Shows the target regions of *C. difficile* used in the claimed invention and oligonucleotide primers for use in detecting *C. difficile* in a sample.

### METHODS

### Bioinformatic analysis of C. difficile sequences

Submitted nucleotide sequences for the two *C*. *difficile* toxin genes *tcdA* and *tcdB* were collated from the National Centre for Biotechnology Information (NCBI) genomic database "GenBank" [http://www.ncbi.nlm.nih.gov/Genbank/] [Accessed 01/10/2008]. In total the nucleotide sequences from 20 toxin A entries and 18 toxin B entries, obtained from unrelated viable strains of *C*. *difficile* were accessed. One further toxin B sequence was obtained from Stabler *et al.* 2008. Conserved regions were pinpointed as described (Rupnik *et al.,* 1999). To confirm that the predicted conserved regions within toxins A and B contained regions of conserved nucleotide sequences, the multiple sequences deposited in the Genebank database were analysed using the Multiple Sequence Alignment (MSA) program ClustalW. The results of this analysis were displayed using Jalview™.

### C. difficile toxin A and B Probe Design

Probes were designed to recognise nucleotide sequences within conserved regions of toxins A and B using Basic Local Alignment Search Tool nucleotide (BLASTn) homology search facility [http://blast.ncbi.nlm.nih.gov/Blast.cgi]. As part of the design process we incorporated features which would enable us to utilise the probes in a future MAMEF assay. The anchor probe for the *C. difficile* assay was designed to be 17 nucleotides in length and to be separated from the 22 nucleotide fluorescent detector probe by a stretch of 5 nucleotides. The anchor probe binds target DNA while the detector probe subsequently binds at a distance which positions the fluorophore at an optimal distance for biomolecular recognition to occur. The use of anchor and detector probes also allows for two levels of sensitivity within the assay for each toxin as the target region is bound at two distinct binding sites. Annealing temperatures of probe and secondary structure analysis was considered, in addition to sequence homology to unrelated targets.

### C. difficile toxin A and B- specific Probe Studies

To enable the designed probes to be tested against a representative collection of *C*. *difficile* isolates, we supplemented a collection of isolates with additional toxinotypes of *C*. *difficile* obtained from the National Anaerobic Reference Unit, Cardiff, Wales, courtesy of Dr. Val Hall. These strains are shown in Table 1. These extra isolates also included clinical isolates from blood culture and variant isolates which only produced toxin B (Ribotypes 017, 047, 110). A further three toxinotypes were tested courtesy of Dr. Katie Solomon, University College Dublin, Ireland. Unless otherwise stated all organisms were stored as spores at 4°C. In total 58 *C. difficile* isolates were tested.

The purity of *C. difficile* strains was confirmed by using *Clostridium difficile* Moxalactam Norfloxacin (CDMN) antibiotic selective supplement. Contents of 1 vial of Oxoid CDMN supplement was reconstituted with 2 ml sdw and added to 500 ml molten (50°C) *C. difficile* agar base together with 7% (v/v) defibrinated horse blood. This was mixed well and poured into sterile Petri dishes, left to dry and degassed.

In addition to increasing the range of *C*. *difficile* isolates tested, the ability of the probes to react with other bacterial species was assessed. Thus to establish the specificity of the designed probes we tested them against a range of bacterial species from closely related *Clostridium* strains, to unrelated strains, as listed in Table 2.

The human gut environment contains approximately 10¹²/g bacteria. Therefore the probes designed to detect *C. difficile* must be able to specifically detect the target regions of the toxin genes amongst the numerous bacteria present. Therefore to further confirm the specificity of the designed probes, metagenomic DNA samples from ten human volunteers were obtained from Cardiff School of Biosciences, Wales, Cardiff, UK, courtesy of Dr. Julian Marchesi. The metagenome was extracted from faecal matter from humans in Zambia, France and the UK.

### DNA Extraction from C. difficile using Chelex 100 Resin

Chelex 100 Resin (BioRad Laboratories, UK) was dispensed into 2 ml sterile distilled water and vortex mixed well using a VortexGenie. Approximately 100 µl Chelex 100 was added to an Eppendorf tube whilst gently agitated. A 10 µl loopful of *C. difficile* colonies from an overnight (24 h) agar plate culture on CDMN was taken and mixed into the Chelex 100. Subsequently the Eppendorfs were placed on a hot plate at 100°C for 12 min, and then centrifuged for 10 min at 15,000 rpm. The supernatant was aliquoted out and stored as DNA extract at -20°C. Concentration of DNA measured using Biophotometer DNA absorbance function calculating absorbance using the relationship that A₂₆₀ of 1.00 = 50 µg/ml pure DNA. DNA purity was calculated at 260 nm/280 nm.

### PCR for C. difficile

As a control a primer known to detect toxin B within *C. difficile* isolates was employed. Only toxin B was detected via PCR as all *C*. *difficile* strains contain a stable toxin B gene.

### Genomic DNA hybridisation dot blots

### Preparation of DIG-labelled Hybridisation Probes

Digoxigenin (DIG) is a non-radioactive molecule with high immunogenicity which is used as an alternative way of labelling oligonucleotides. The probes were prepared for dot blot hybridisation by performing a PCR reaction by replacing standard dNTPs with DIG-labelled dNTPs in a chemiluminescence-based method.

To allow all of the *C. difficile* isolates to be examined easily via dot blot, a Macro-arraying technique was employed to hybridise gDNA samples onto the positively charged nylon membrane. The gDNA extracted from the *C. difficile* isolates (using Chelex 100), was aliquoted at 30 µl per well into a 384 well plate and printed into the positively charged membrane using a Flexys robotic workstation. The robot was set to blot 4 spots of DNA in a grid format (Figure 1). To orientate the membrane a single well was included, containing loading dye and 50 ng/ µl lambda phage. After macroarraying the gDNA was fixed to the nylon membrane using a UV transilluminator for 5 min. The fixed membranes were stored for future use between two sheets of blotting paper at room temperature.

### DNA hybridisation dot blots

To prepare the membrane for hybridisation the membrane was calibrated in a pre-hybridisation step. The hybridisation tubes were washed thoroughly and the hybridisation oven warmed to 50°C beforehand. For pre-hybridisation, 20 ml of DIG Easy Hyb buffer was put in each hybridisation tube and left in the hybridisation oven until they reached 50°C. Subsequently the membrane was put into the hybridisation tube with DNA side facing the tube interior and a further 20 ml heated DIG Easy Hyb buffer was added to give 40 ml in each tube.

For hybridisation of the DIG labelled probes to any DNA on the membranes, another hybridisation step was undertaken. Approximately 5-10 µl of the DIG-labelled PCR product probe to 500 µl of DIG Easy Hyb buffer and boiled in a beaker on a hot plate for 10 min (98°C) followed by chilling immediately on ice. The boiled probe was added to the pre-heated hybridisation tube after Prehybridisation stage and subsequently the tubes were rotated in the hybridisation oven overnight at 50°C.

The stringency washes were employed to remove any unbound probe and carried out at approximately 10°C above the hybridisation temperature. The membrane was washed in 40 ml 2x SSC stringency solution twice for 15 min, and then in 40 ml 0.5x SSC stringency solution twice for 25 min. For high stringency the membrane was washed in 40 ml 0.1x SSC stringency solution twice for 15 min.

### Detection of bound probe using DIG chemiluminescence

The bound probes were detected on the membrane using an anti-DIG alkaline phosphatase method. The membrane was equilibrated in washing buffer for 2 min then agitated in 1% blocking solution for 45 min. The membrane was then agitated for 30 min in a 1:10,000 dilution of anti-DIG alkaline phosphatase antibody in 1 % blocking solution.

The membrane was then washed twice for 15 min in washing buffer to remove any unbound antibody from the membrane, and equilibrated in detection buffer for 2 min. In an eppendorf 10 µl CSPD was added to 1000 µl detection buffer. The membrane was placed DNA side up onto a clean plastic bag and the 1000 µl CSPD pipetted over the membrane. The membrane was incubated at 37°C for 15 min. and the blots were exposed overnight.

Blots were imaged with a chemiluminescent camera and visualised using VisionWorks® LS analysis software (UVP, UK).

### Bacterial Strains, biological fluids and genomic DNA

Genomic DNA was isolated from *C. difficile* strain CD630 using Chelex 100 resin. Whole milk (3.5g fat content) was purchased from Whole Foods (Harbour East, Baltimore, MD, USA). PBS (0.01 M phosphate buffer, 0.0027 M potassium chloride and 0.137 M sodium chloride) was made by dissolving 1 tablet in 200 ml diH₂O (Sigma Aldrich, USA). Human faecal matter was provided by a healthy volunteer.

### Anchor and Fluorescent Probes and target DNA

Probes specific for regions of toxin A and toxin B of *C. difficile* were modified to enable incorporation into the MAMEF detection platform (Figure 2; Table 3). A thiol group was added to the 5' region of the anchor probe to enable binding of the DNA to the surface of the silver island film (SiF) while the capture probe was labelled with an Alexa® fluorophore. The Alexa fluorophore 488 (green) was used to label toxin A and the Alexa fluorophore 594 (red) was used to label toxin B.

### Formation of gold lysing triangles to microwave C. difficile

To focus the microwave power in the microwave, gold lysing triangles were used. Gold lysing triangles were provided by the Institute of Fluorescence (University of Maryland). Glass microscope slides (Starfrost®, LightLabs, USA) were covered with a paper mask (12.5 mm in size and 1 mm gap size) leaving a triangle bow-tie region exposed. Gold was deposited onto the glass microscope slides using a BOC Edwards 306 Auto vacuum E-beam evaporation deposition unit, in equilateral gold triangles of 12.5 mm and 100 nm thick at 3.0 x 10⁻⁶ Torr (Tennant *et al.,* 2011). Two layers of self-adhesive silicon isolators (Sigma Aldrich, USA) (diameter = 2.5 mm) were placed on top of the bow-tie regions, creating a lysing chamber (Zhang *et al.,* 2011).

### The release of C. difficile DNA from vegetative cells and spores using microwave irradiation

To optimise DNA release the following approaches were investigated:
**(i) Boiling** *C*. *difficile* (1 x 10⁵ cfu/ml) was suspended into PBS buffer into 15 ml Falcon tubes and boiled in a water bath on a hot plate for 3 h. After each 30 min, a 100 µl sample was taken from the boiling spores and cooled on ice. The DNA released from *C. difficile* was examined using gel electrophoresis.
**(ii) Gold Triangles** *C*. *difficile* (1 x 10⁵ cfu/ml) was suspended into PBS buffer and pipetted at a volume of 500 µl into the gold tie lysing chamber and exposed to a 15 s microwave pulse at 80% power in a GE microwave Model No. JE2160BF01, kW 1.65 (M/W). The microwaved solution was examined for the presence of viable organisms. Further experiments involved spiking milk and human faecal matter (both diluted with PBS buffer) with varying concentrations of *C*. *difficile* and then performing microwave radiation. Each experiment was conducted in triplicate.

### Bacterial quantification after focussed microwave irradiation

**(i)Vegetative cells:** The control samples (4 × 10⁶ cfu/ml) were serially diluted using Miles Misra (1938) drop count method. Briefly 20 µl of bacterial sample was serially diluted in 1:10 ratio in 180 µl BHI broth and drop counts performed on BHI agar and incubated at 37°C in a 3.4L anaerobic jar with an anaerobic gas generating kit. Post lysis the number of remaining viable bacteria (cfu/ml) was enumerated using the Miles Misra method as above (1938).
**(ii) Spores:** The control samples (1.33 x 10⁷ cfu/ml) were serially diluted using Miles Misra (1938) drop count method. Briefly 20 µl of the spore sample was serially diluted in 1:10 ratio in 180 µl BHI broth and drop counts performed on BHI agar supplemented with the spore germinant sodium taurocholate (0.1%). Plates were incubated at 37°C in a 3.4L anaerobic jar with an anaerobic gas generating kit. Before DNA release the sample was heated at 80°C for 10 min to ensure removal of any vegetative cells. After lysis the number of remaining viable spores (cfu/ml) was enumerated using the Miles- Misra method as above (1938).

### Silver Island Film formation on glass substrate

Silver island Films (SiFs) were prepared on Silane-prep ™ glass slides to enable silver adhesion. A solution of silver nitrate was made by adding 0.5 g in 60 ml dH₂O to 200 ml of freshly prepared 5% (w/v) sodium hydroxide solution and 2 ml ammonium hydroxide. The solution was continuously stirred at RT, and then cooled to 5°C in an ice bath. The slides were soaked in the solution and 15 ml of fresh D-glucose was added (0.72 g in 15 ml dH₂O). The temperature of the solution was raised to 40 °C and as the colour of the mixture turned from yellow/green to yellow-brown, the slides were removed from the mixture, washed with dH₂O, and sonicated using an MSE Soniprep 150 sonicator for 1 min at RT. SiFs used in this study were between an OD₄₅₀ of 0.4 - 0.5.

### Preparation of MAMEF assay platform for detection of C. difficile DNA

Glass slides with SiFs deposited were coated with self-adhesive silicon isolators containing oval wells (2.0 mm=diameter, 632 mm=length, 619 mm=width). The thiolated anchor probe was decapped to remove the thiol groups and enable binding to the SiF. This was achieved by diluting 40 µM anchor probe into 100 µl of 1 M Tris-EDTA (TE) buffer and adding 9 µl to 250 mM of 20 µl dithiothreitol (DTT) (38.5 mg DTT into 1 ml TE buffer). The mixture was incubated at RT for 60 min. The decapped anchor probe (1 µM) was diluted into 4 ml TE buffer and 100 µl of anchor probe was added to and incubated in each oval well of the SiF for 75 min. After incubation the anchor was removed and 50 µl of 1 µM Alexa-Fluor® probe was added to 50 µl of DNA from the target organism. The SiFs containing the bound probes and DNA was then incubated using MAMEF for 25 s in a microwave cavity at 20% microwave power, GE microwave Model No. JE2160BF01, 1.65 kiloWatts. In the presence of target DNA the three piece assay is complete and enhanced fluorescence can be observed. The sample was then removed from the well after MAMEF and the well washed with 100 µl TE buffer 3 times.

### Detection and fluorescence spectroscopy

The presence of target DNA was confirmed by the generation of a fluorescent signal following excitation with laser light. The following fluorophores were employed. Fluorescence was emitted by the DNA MAMEF capture assay and measured using a diode laser and a Fibre Optic Spectrometer (HD2000) by collecting the emission intensity (I) through a notch filter.

### Results

### Bioinformatic analysis of C. difficile sequences

Conserved nucleotide regions within the sequences of toxins A and B were identified by employing Rupnik's toxin typing method (1998). These conserved sequences lacked restriction sites and we employed bioinformatic analysis to design probes specific to toxins A and B from these regions. Conserved regions from different clinical isolates (20 from *tcdA* and 19 from *tcdB*) of *C*. *difficile* were collated from the Genbank database and imported into ClustalW to identify common areas from which to design probes. Two conserved regions within toxin A and four conserved regions within toxin B were identified (Table 4). These results confirmed that the regions defined by Rupnik *et al.* (1998) were indeed conserved within both *C. difficile* toxins, and these conserved regions were suitable regions to investigate for probe design for the MAMEF- based assay.

### Probe Design

Probes were designed to recognise sequences within each conserved region (∼50 nucleotides; Table 4). For the MAMEF assay, ideally anchor probes were 17 nucleotides in length while the fluorescent detector probes were ideally 22 nucleotides in length, and a 5 nucleotide gap between the anchor and fluorescent probes was incorporated to fit our MAMEF assay requirements. The potential capture and fluorescent detector probes designed from the regions can be seen in Tables 4, 5 and 6. However, only those which showed homology to *C*. *difficile* only were selected for commercial synthesis (Table 7).

### Sensitivity of DIG-labelled probes

The sensitivities of each DIG-labelled DNA probe were assessed to determine the optimal concentration for use in further dot blot experiments. The probe with the highest sensitivity and strongest chemiluminescent signal was produced by the *tcdA* 76 bp Anchor probe, which gave a signal at a dilution of 10 ng/µl (figure 3). This is important for capturing target DNA within a future MAMEF based assay. On the basis of the sensitivity results the concentration of probe used for subsequent macroarray screening studies was 60 ng/ µl.

### Macro-arraying genomic DNA onto a positively charged nylon membrane of C. difficile isolates

Genomic DNA from the *C. difficile* isolates tested were macroarrayed as shown in Table 8. As expected each strain containing a copy of the toxin A and B gene sequences gave a positive signal, the strength of which varied between isolates, likely due to an artefact of experimental procedure.

To confirm the specificity of the probes variant isolates of *C*. *difficile* (*tcdA*⁻*tcdB*⁺) lacking either the toxin A (ribotypes 017; 047: *tcdA*⁻*tcdB*⁺) or toxin B (Toxinotype Xla; Xlb; DS1684: *tcdA⁻tcdB⁻*) gene sequences were included in the panel. The DNA from these isolates did not bind to the probes (figure 4).

To further confirm the specificity of the probes genomic DNA from other bacterial species, both close and distant relatives were subject to hybridisation analysis (figure 5). The probes did not bind to the bacterial species unrelated to *C. difficile* further indicating that the probes were highly specific to toxins A and B of *C. difficile.* Species of the *Clostridium* genus, including species of the LCT family closely related to *C. difficile* (which possess toxins closely related to *tcdB*) did not shown any probe hybridisation which further validates the specificity of the probes.

Additionally, metagenomic DNA from human gut flora of ten human volunteers was examined for hybridisation against the designed DNA probes (Table 2). The probes did not bind any human metagenomic DNA samples. The *tcdA* 76bp anchor and detector probes, and the *tcdB* anchor and detector probes were used in further analysis.

### Detection of synthetic DNA targets by MAMEF

The ability of the toxin A and B probes to detect synthetic target DNA (43 nucleotides in length) was determined. Target DNA was diluted in TE buffer to give the following range of concentrations; 1 nM, 10 nM, 100 nM, 500 nM, 1000 nM and 100 µM with TE buffer serving as a control. The lowest concentration of DNA detected by each probe was 1 nM (Figure 6A & C). Targets were readily detected by MAMEF as can be seen in Figure 6B and D. For toxin A probes the excitation is 495 nm, and emission: 519 nm, and toxin B excitation is 590 nm and emission is 617 nm.

### Detection of synthetic DNA in whole milk by MAMEF

Target DNA was mixed with whole milk. The whole milk was diluted by 10% with PBS and the synthetic target DNA was diluted in the milk to final concentrations of 1 nM, 10 nM, 100 nM, 500 nM and 1000 nM. As can be seen in Figure 7B and D the limit of sensitivity was similar to that seen with PBS at 1nM for both Toxin A and B. Interestingly reducing the concentration of Toxin B anchor probe from 10 µM to 1 µM did not affect the sensitivity of the assay. As before there was an increase in fluorescent intensity as the concentration of the target oligonucleotide increased although the intensity of the signal was less (∼5 AU) than that seen in the presence of PBS when comparing the results from experiments using 1 µM anchor (Figure 6 A; Figure 7A & C). At the concentration of 10 nM the intensity in the presence of milk for Toxin A is -20 AU and Toxin B is 25 AU.

### Detection of synthetic DNA in human faecal matter by MAMEF

Target DNA was mixed with human faecal matter. Faecal matter was diluted by 50% with PBS and the synthetic target DNA was then added to give the following range of concentrations; 1 nM, 10 nM, 100 nM, 500 nM and 1000 nM. The limit of sensitivity was 1 nM for toxin A and 1 nM for toxin B. As expected an increase in fluorescent intensity was observed as the concentration of target oligonucleotide increased (Figure 8A & B). The signal intensity however was less than observed in the presence of PBS and whole milk for both toxins A and B (Figure 6 A; Figure 7A & C; Figure 8A & B). At the concentration of 10 nM the intensity in the presence of milk for Toxin A is -10 AU and Toxin B is 12 AU which are similar.

### Statistical analysis of synthetic DNA detection within organic matrices

Statistical analysis of differences in synthetic target DNA detection of toxin A was performed using one way ANOVA, which revealed a P value of 0.0534. Thus as P > 0.05, this indicates there is no significant difference between the organic matrices used (TE buffer, Milk and faecal matter). This was further confirmed using the Kruskall-Wallis test where P = 0.2588.

One way ANOVA results of toxin B synthetic target DNA detection revealed a P value of 0.107. As P > 0.05 there is no significant difference between the organic matrices used (TE buffer, milk and faecal matter). This was further confirmed using the Kruskall-Wallis test where P = 0.158. Also there was a significant difference between the control sample of toxin A and B and the test concentrations as determined by Dunnetts multiple comparison of variance test (Graphpad Prism 5).

### Detection of genomic DNA from C. difficile Strain 630

To establish if the toxin A and B probes could detect targets within gDNA, we employed DNA from *C. difficile* strain CD630. Genomic DNA from CD630 was microwaved in PBS buffer for a shorter time of 8 seconds at 70% power to determine if target DNA could be detected. A control of unmicrowaved gDNA in PBS was employed. The toxin probes were able to detect gDNA at concentrations of 0.010 µg before microwave treatment, and 0.010 µg and 0.005 µg after microwave treatment (Figure 9A & B). The limit of sensitivity was 0.005 µg gDNA for both toxin A and toxin B. There is an increase in fluorescent intensity as the concentration of the oligonucleotide increases (Figure 9A & B). The signal intensity however was similar to synthetic target intensity observed in the presence of PBS, and greater than that observed when the synthetic targets were tested in the presence of whole milk and faecal matter (Figure 6 A; Figure 7A & C; Figure 8).

### Release of target DNA from a C. difficile spore preparation via boiling

A boiling based method was developed to release DNA from *C*. *difficile.* The release of DNA from spores was assessed by mixing spores from a ∼80% preparation with either PBS or diH₂0, followed by boiling for 3 hours with samples removed every 30 min to determine if DNA has been released using gel electrophoresis (figure 10). As can be seen in Figure 5.8, there appeared to be no DNA release from water-treated spores. However in contrast, spores suspended in PBS produced a smear upon gel electrophoresis which was thought to represent fragmented DNA. Therefore PBS was used as the diluent for samples used in further experiments.

### Release of DNA from a C. difficile spore preparation using focussed microwave irradiation

We sought to develop a method which would rapidly release target DNA using microwave radiation. Gold triangles were used to assist in breaking open bacteria when exposed to microwave irradiation. This method has previously been used with *B. anthracis* to release DNA from spores (Aslan *et al,* 2008). Rapid heating occurs at the 1 mm gap between two adjacent triangles where the microwaves are focussed thus reducing the need for complex DNA extraction methods. A total of 500 µl of vegetative bacteria and spores were tested at a range of microwave powers and times until an optimal period of irradiation was identified (15 s in the microwave cavity at 80% power). Each sample was run under gel electrophoresis to establish DNA release (Figure 11).

### Bacterial quantification following focussed microwave irradiation

The number of viable vegetative bacteria and spores in our spore preparations pre-and post- microwave treatment were determined (Figure 12). We observed a 4 x 10³ cfu/ml log reduction in vegetative counts following microwave treatment. The corresponding reduction in viable spores was 1.33 x 10⁴ cfu/ml.

### Detection of C. difficile target DNA in PBS

Using the conditions described above (80% microwave power) spores and vegetative cells of *C*. *difficile* CD630 were suspended in PBS and microwaved for 15 s. Following treatment, vegetative bacteria were diluted to give the following range of final concentrations in each assay well; 1000 cfu, 100 cfu and 10 cfu of vegetative bacteria. Following treatment, spores were diluted to give the following range of final concentrations in each assay well; 10000 cfu, 1000 cfu, 100 cfu and 10 cfu. PBS buffer was used as a control (Figure 13A & B).

The probes readily detected DNA released from vegetative cells in PBS, yielding a signal of approximately 20 Intensity units at 1000 cfu. The limit of sensitivity was 10 cfu for both toxin A and toxin B. There is an increase in fluorescent intensity as the concentration of vegetative cells increases (Figure 13 A). However the signal intensity was higher for the toxin B probe at 60 Intensity Units at 1000 cfu (Figure 13B). As mentioned previously this may be due to a range of factors including anchor concentration, optical density of the SiF and efficiency of the fluorophore emission upon binding to target DNA.

The probes readily detected DNA released from the spore preparation in PBS, yielding a strong signal of approximately 15 Intensity units at 1000 cfu. The limit of sensitivity was 10 cfu for both toxin A and toxin B. There is an increase in fluorescent intensity as the concentration of spore preparation increases (Figure 14 A). The signal intensity is similar for the toxin B probe at 18 Intensity Units at 1000 cfu (Figure 14 B).

### Detection of C. difficile target DNA in whole milk

Spores of *C. difficile* CD630 were suspended in milk and microwaved for 15s using 80% microwave power. Following treatment, spores were diluted to give the following range of final concentrations in each assay well; 1000 cfu, 100 cfu and 10 cfu (Figure 15). The probes readily detected DNA released from spores in milk and the intensities appear to be similar as can be seen in Figure 15A & B. The limit of sensitivity was 10 cfu for both toxin A and toxin B. For the concentration of 1000 cfu the signal intensity was ∼ 5AU less than in the presence of PBS for both toxins A and B (Figure 15A & B). However the signal is higher for the toxin B probe when detecting the spores (25 Intensity Units at 1000 cfu) in Figure 15B compared to the same concentration in toxin A.

### Detection of C. difficile target DNA in Human faecal matter

Spores of *C. difficile* CD630 were suspended in human faecal matter and microwaved for 15s using 80% microwave power. Following treatment, spores were diluted to give the following range of final concentrations in each assay well; 1000 cfu, 100 cfu and 10 cfu (Figure 16). The probes readily detected DNA released from spores in faeces as can be seen in Figure 16A & B. The limit of sensitivity was 10 cfu for both toxin A and toxin B. The signal intensity was slightly higher than observed in the presence of PBS and milk at 25 AU for toxin A and 20 AU for toxin B (Figure 16A & B; Figure 16A & B). In this experiment toxin A emitted a higher fluorescent signal than toxin B in response to the target.

### Statistical analysis of C. difficile DNA detection within organic matrices

Statistical analysis of toxin A target DNA detection was performed using one way ANOVA, in conjunction with a Kruskall-Wallis test revealing a P value of 0.069. Thus as P >0.05, this indicates there is no significant difference between the organic matrices used (TE buffer, Milk and faecal matter). One way ANOVA of toxin B target DNA detection revealed a P value of 0.008. As P < 0.05 there is a significant difference in detection between the organic matrices used (TE buffer, Milk and faecal matter). This was further confirmed using the Kruskall-Wallis test where P = 0.018. There is no significant difference in detection between spore concentrations used; however there was a significant difference between the control samples of tcdA and B and the test concentrations as determined by Dunnetts multiple comparison of variance test (Graphpad Prism 5).

### Summary

The main findings of this study can be summarised as follows:
Using a combination of bioinformatics and sequence analysis of a large panel of clinical *C*. *difficile isolates,* conserved target regions were found to exist in the genes encoding the pathogenic toxins (*tcdA* and *tcdB*)*.* Using these specific conserved target regions, we designed complimentary binding oligonucleotide probes to detect these specific regions. Through an extensive screening process, it was found that selected oligonucleotide probes specifically detect and distinguish the *tcdA and tcdB* target regions. It was found that these probes were highly specific with a high level of sensitivity, with the ability to detect the target regions from a range of *C. difficile* isolates. Furthermore, these specific oligonucleotide probes did not cross-react with isolates from related *Clostridium* strains, other bacterial isolates, or against human gut metagenomic DNA extracts. Consequently, we have therefore identified highly conserved and specific target regions which can accurately be detected to diagnose or identify the presence of *C*. *difficile* in a sample. Moreover, these regions and their corresponding probes permit discrimination of different toxinotypes of *C. difficile* strains with the ability to detect the genes encoding both toxins A and/or B.

As a proof of concept, we investigated the detection of these conserved target regions identified from *C*. *difficile* Toxin A and Toxin B using a MAMEF detector platform. By doing so, we were able determine the ability and sensitivity of these conserved target regions/their corresponding oligonucleotides using MAMEF to identify *C. difficile* target DNA in a range of organic matrices. We were able to detect as few as 10 cfu of *C*. *difficile* in a faecal suspension within 40 seconds. To put this into context, this represents a high level of sensitivity as in human infection approximately 10⁶- 10⁸ spores are released. This level of sensitivity and speed compares favourably with all of the currently available *C. difficile* detection methods. Indeed, there is presently no assay detecting *C. difficile* directly within faecal samples. As far as we are aware, our assay is also the only system capable of detecting both toxins A and B.

We have therefore identified highly conserved target regions of *C*. *difficile,* which can be detected to provide a highly sensitive and accurate test for the presence of the organism. Detection of these specific conserved target regions therefore transforms the sensitivity for the identification of *C. Difficile,* including strains and toxinotypes thereof, in a sample.

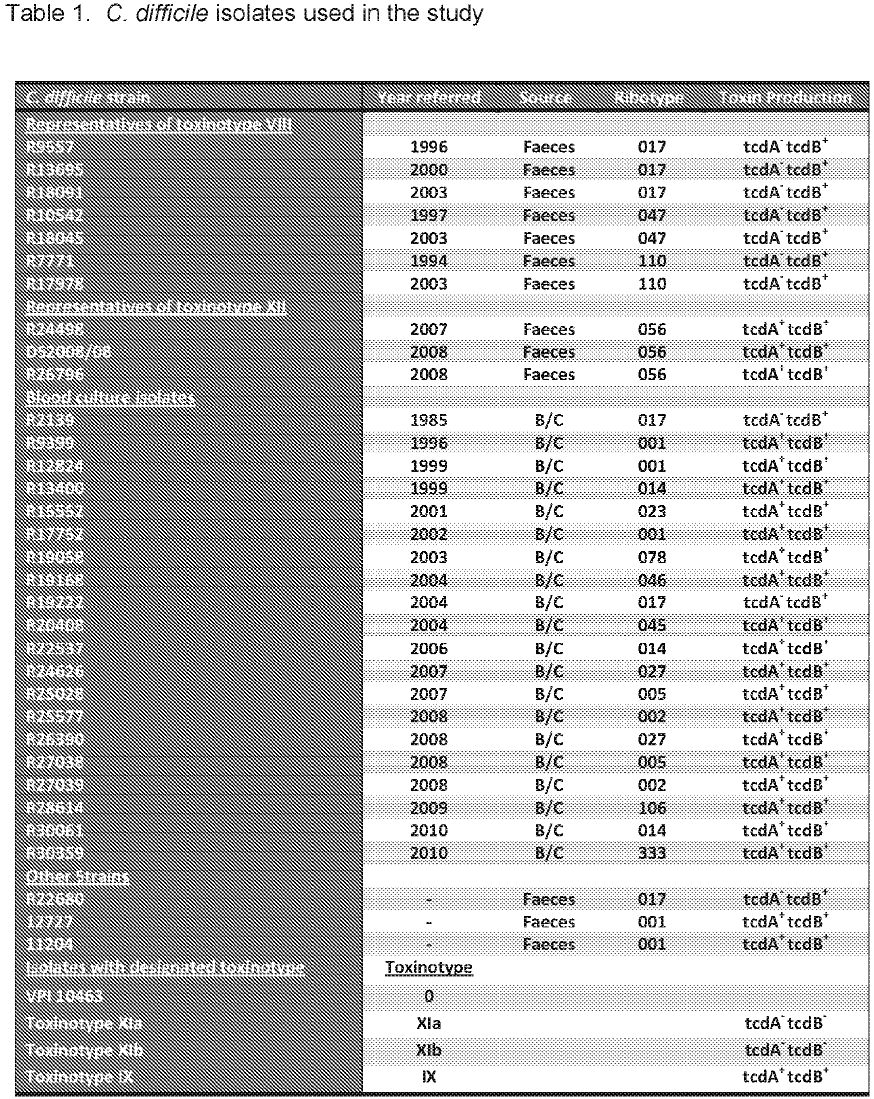

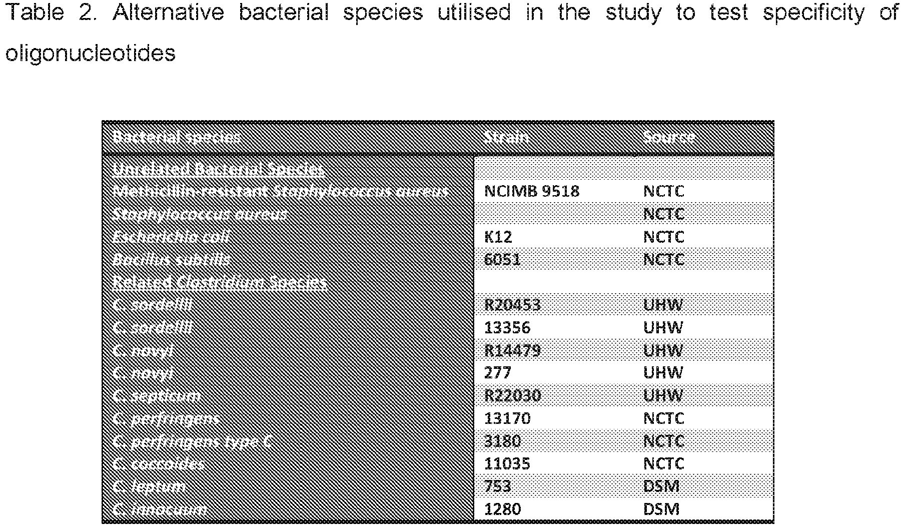

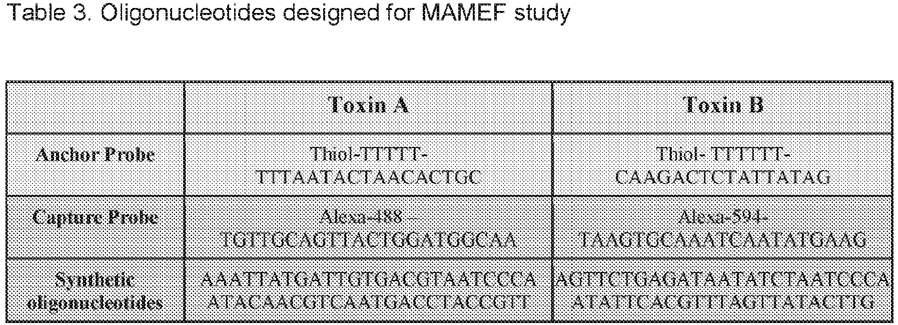

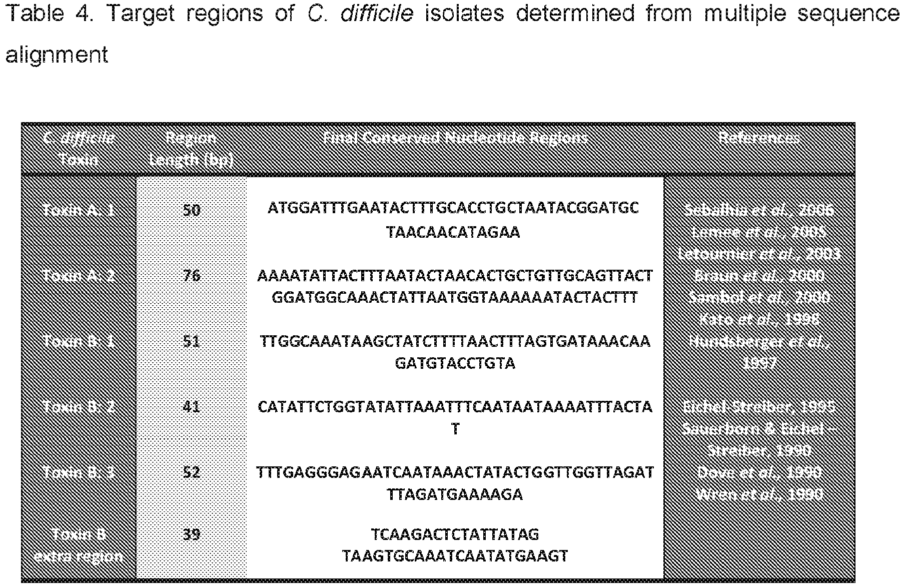

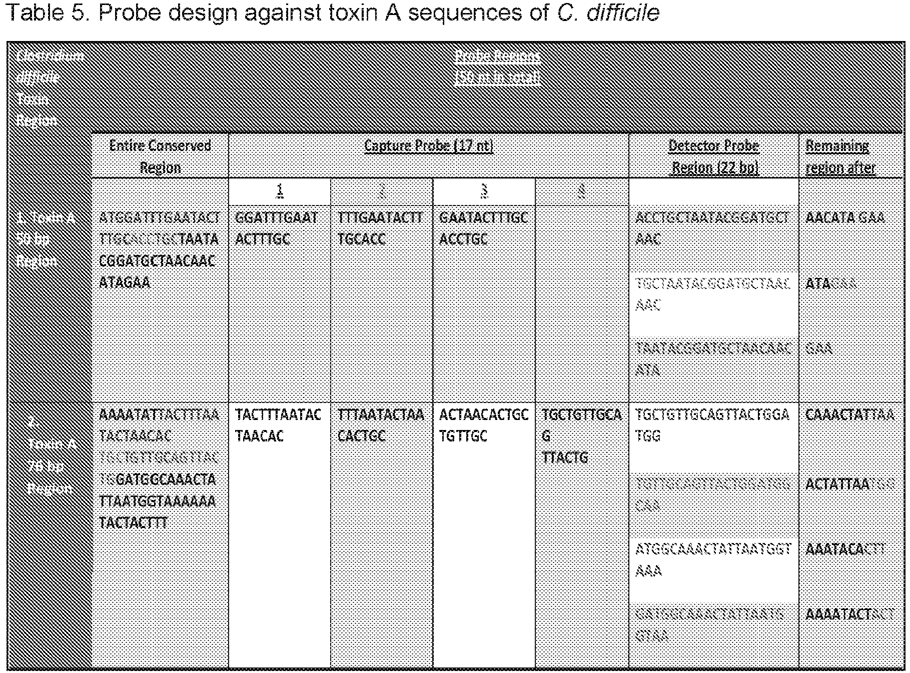

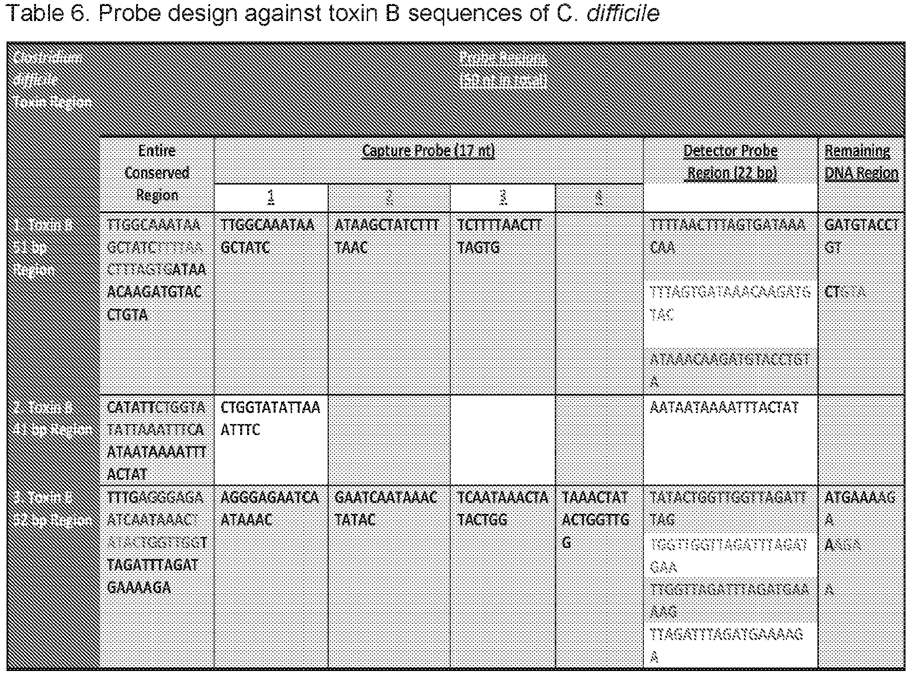

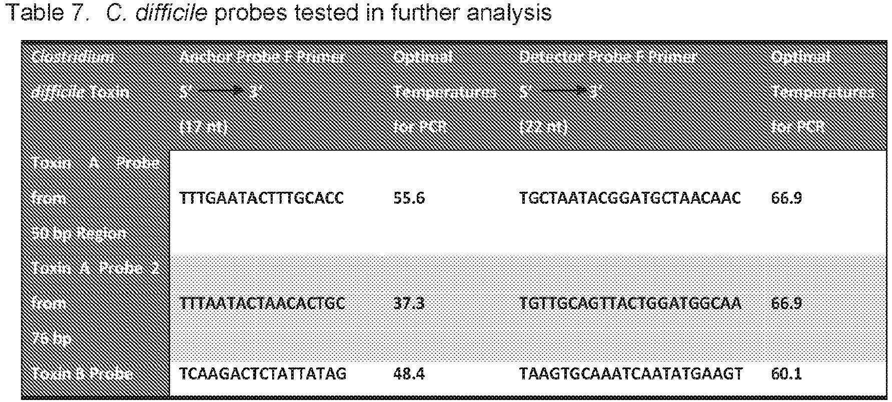

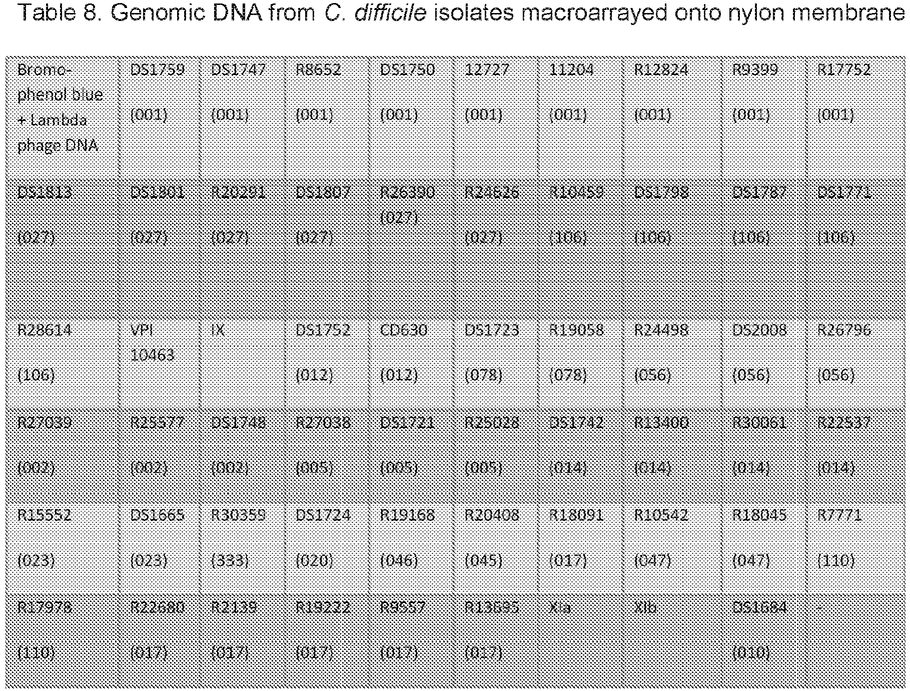

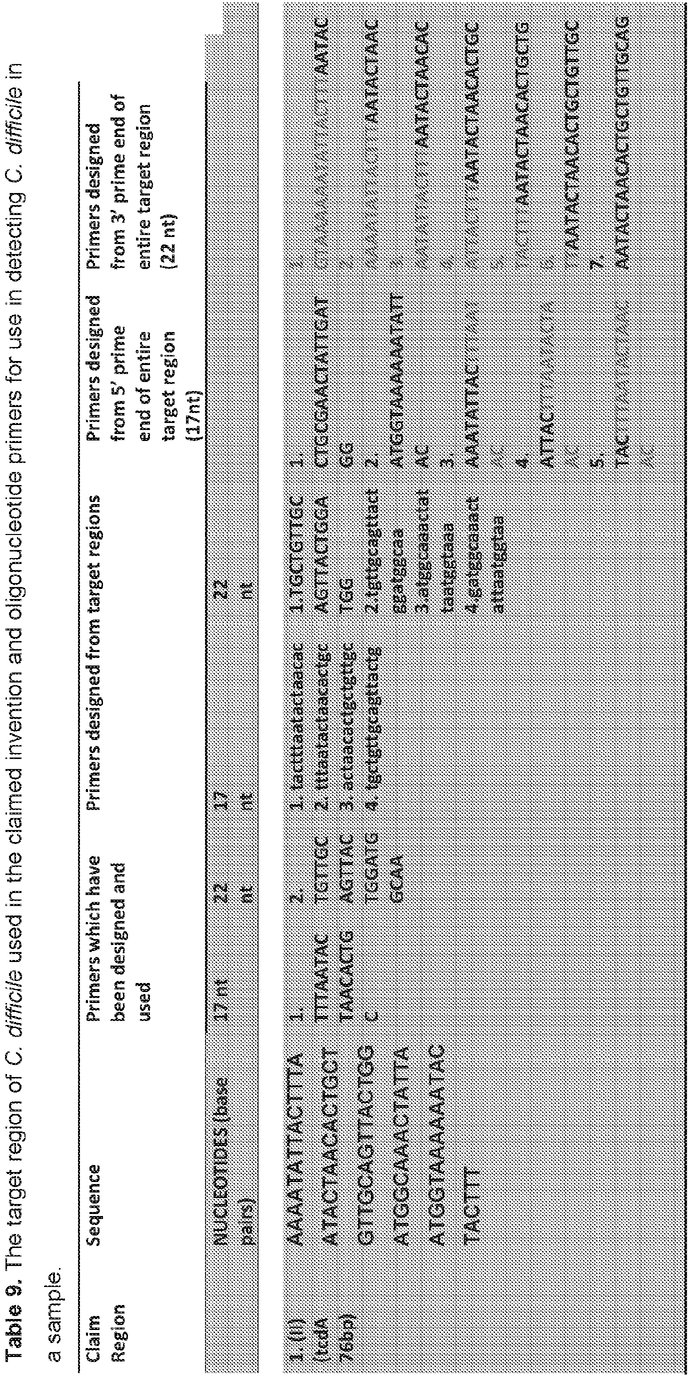

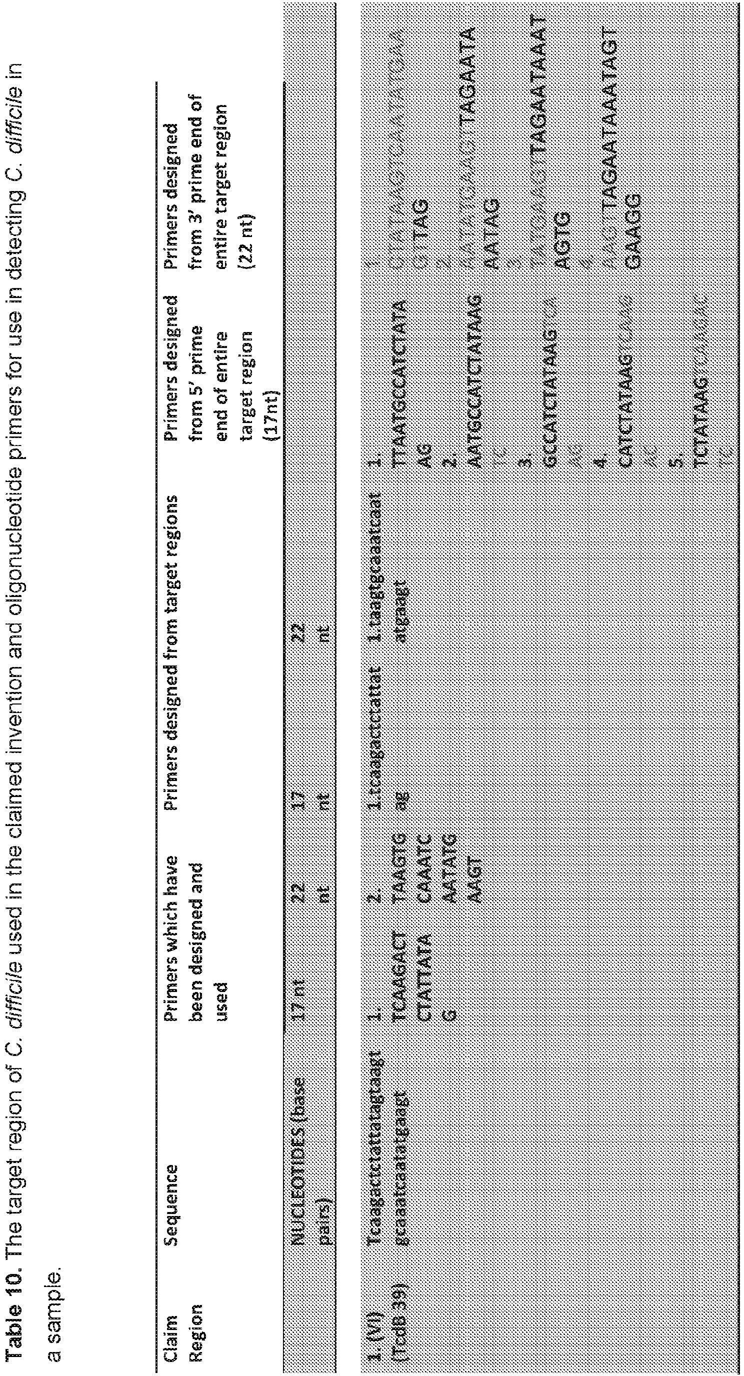

### References

Asian, K., Previte, M.J., Zhang, Y. & Geddes, C.D. (2008) Microwave-accelerated surface plasmon-coupled directional luminescence 2: a platform technology for ultra fast and sensitive target DNA detection in whole blood. J Immunol Methods 29;331(1-2)-103-13.
Rupnik, M., Avesani, V., Janc, M., von Eichel-Streiber, C. & Delmee, M. (1998) A Novel Toxinotyping Scheme and Correlation of Toxinotypes with Serogroups of Clostridium difficile Isolates. J Clin Microbiol. 36(8): 2240-2247.
Tennant, S.M., Zhang, Y., Galen, J.E., Geddes, C.D. & Levine M.M. (2011) Ultra-fast and sensitive detection of non-typhoidal Salmonella using microwave-accelerated metal-enhanced fluorescence ("MAMEF"). PLoS One 8;6(4)e18700.
Zhang, Y., Agreda, P., Kelley, S., Gaydos, C. & Geddes, C.D. (2011) Development of a microwave-accelerated metal-enhanced fluorescence 40 second, <100 cfu/ml point of care assay for the detection of Chlamydia trachomatis. IEEE Trans Biomed Eng. 58(3):781 -4.

## Claims

1. A method for the detection of *C. difficile* in a sample wherein said *C. difficile* comprises a nucleic acid target region having the following sequence:
I) Tcaagactctattatagtaagtgcaaatcaatatgaagt;
which method comprises exposing said sample to:
a) at least one oligonucleotide probe that is complementary to at least 15 nucleotides of said target region, or;
b) an oligonucleotide probe that is at least 80% homologous to the oligonucleotide in a); and
detecting the binding of said oligonucleotide to said target region and, where binding occurs, concluding that *C. difficile* is present in said sample.

2. The method according to claim 1 wherein a further test is undertaken on said sample, sequentially or simultaneously, with the method of claim 1 to determine if a further nucleic acid target region having the following sequence is present:
II) Aaaatattactttaatactaacactgctgttgcagttactggatggcaaactattaatggtaaaaaatactacttt; which method comprises exposing said sample to:
d) at least one oligonucleotide probe that is complementary to at least a part of said further nucleic acid target region, or;
e) an oligonucleotide probe that is at least 80% homologous to the oligonucleotides in d); and
detecting the binding of said oligonucleotide to said further nucleic acid target region and, where binding occurs, concluding that *C. difficile* is present in said sample.

3. The method according to any preceding claim wherein said sample is selected from the group comprising: earth; soil; faeces; urine; body fluid; food; laboratory cultures; hospital equipment; or wound dressings.

4. The method according to any preceding claim wherein DNA is extracted from said sample.

5. The method according to any preceding claim wherein said oligonucleotide is designed for use in an oligonucleotide binding assay selected from the group comprising: PCR, Southern Blotting, DNA dot blotting, DNA microarray techniques, Microwave-assisted annealing assays.

6. The method according to any preceding claim wherein said oligonucleotide comprises:
a signalling molecule which emits a signal when said oligonucleotide exists in either a bound or an unbound state; or emits a quantitative signal whose scale is representative of at least one of said bound or unbound states.

7. The method according to any preceding claim wherein said oligonucleotide comprises a part of a signalling system which part interacts with other parts of said system to emit a signal when either in a bound state or an unbound state whereby the binding of said oligonucleotide to said target region can be detected.

8. The method according to any one of claims 6-7 wherein said signalling molecule may be selected from the group comprising: a fluorescent molecule; a chemiluminescent molecule; or a bioluminescent molecule.

9. The method according to any preceding claim wherein said oligonucleotide probe comprises a pair of probes including an anchor probe and a labelled detector probe.

10. The method according to claim 9 wherein said anchor probe binds a site separated by between 1-20 nucleotides from the site bound by said detector probe.

11. The method according to claim 9 or 10 wherein said anchor probe binds a site separated by 5 nucleotides from the site bound by said detector probe.

12. The method according to any one of claim 9-11 wherein said anchor probe further comprises a chemical group/molecule for attaching, or adhering, said probe to a surface.

13. The method according to claim 12 wherein said chemical group/molecule is a thiol group or biotin.

14. The method according to claim 12 or 13 wherein said anchor probe further comprises at least one T nucleotide base between said chemical group/molecule and a binding nucleotide of said anchor probe to said target region.

15. The method according to any preceding claim comprising a plurality of oligonucleotide probes labelled with the same or different signalling molecules.

16. The method according to any preceding claim wherein the concentration of said oligonucleotide probes is between 10-100 ng/ul.

17. The method according to any preceding claim wherein said probes are selected from the group comprising:
between 15 - 30 nucleotides or 15 - 25 nucleotides or 17 - 22 nucleotides in length.

18. The method according to any preceding claim wherein said oligonucleotide is/are selected from the group comprising:
tcaagactctattatag; and taagtgcaaatcaatatgaagt.

19. The method according to any preceding claim wherein said oligonucleotide is:
ctataagtcaatatgaagttag.

20. The method according to any one of claims 2-19 wherein said oligonucleotides are selected from the group comprising:
tactttaatactaacac; tttaatactaacactgc; actaacactgctgttgc; tgctgttgcagttactg; tgctgttgcagttactggatgg; tgttgcagttactggatggcaa; atggcaaactattaatggtaaa; and gatggcaaactattaatggtaa.

21. The method according to any one of claims 2-19 wherein said oligonucleotides are selected from the group comprising:
ctgcgaactattgatgg; atggtaaaaaatattac; aaatattactttaatac; attactttaatactaac; tactttaatactaacac; gtaaaaaatattactttaatac; aaaatattactttaatactaac; aatattactttaatactaacac; attactttaatactaacactgc; tactttaatactaacactgctg; ttaatactaacactgctgttgc; and aatactaacactgctgttgcag

22. The method according to any preceding claim wherein said method is a diagnostic method for identifying individuals suffering from a *C. difficile* infection.

23. A kit for the detection of *C. difficile* in a sample wherein said *C. difficile* comprises a nucleic acid target region having the following sequence:
I) Tcaagactctattatagtaagtgcaaatcaatatgaagt;
which kit comprises:
a) at least one oligonucleotide probe that is complementary to at least 15 nucleotides of said target region; or ;
b) an oligonucleotide probe that is at least 84% homologous to the oligonucleotide in a); and
optionally, reagents and/or instructions for practising said detection.

24. The kit according to claim 23 wherein said *C. difficile* further comprises a nucleic acid target region having the following sequence:
II) Aaaatattactttaatactaacactgctgttgcagttactggatggcaaactattaatggtaaaaaatactacttt; and which kit further comprises:
d) at least one oligonucleotide probe that is complementary to at least a part of said further nucleic acid target region, or;
e) an oligonucleotide probe that is at least 80% homologous to the oligonucleotides in d); and
detecting the binding of said oligonucleotide to said further nucleic acid target region and, where binding occurs, concluding that *C. difficile* is present in said sample.

25. The kit according to claim 23 or 24 wherein said oligonucleotide comprises:
a signalling molecule which emits a signal when said oligonucleotide exists in either a bound or an unbound state; or emits a quantitative signal whose scale is representative of at least one of said states.

26. The kit according to any one of claims 23 - 25 wherein said oligonucleotide comprises a part of a signalling system which part interacts with other parts of said system to emit a signal when either in a bound state or an unbound state whereby the binding of said oligonucleotide to said target site can be detected.

27. The kit according to any one of claim 23 - 26 wherein said oligonucleotide probe comprises a pair of probes including an anchor probe and a labelled detector probe.

28. The kit according to claim 27 wherein said anchor probe further comprises a chemical group/molecule for attaching, or adhering, said probe to a selected surface.

29. The kit according to claim 28 wherein said anchor probe further comprises at least one T nucleotide base between said chemical group/molecule and a binding nucleotide of said anchor probe to said target region.

30. The kit according to any one of claims 23 - 29 wherein said kit comprises at least one oligonucleotides selected from the group comprising:
tcaagactctattatag; and taagtgcaaatcaatatgaagt.

31. The kit according to any one of claims 23 - 29 wherein said kit comprises at least one oligonucleotides which is:
ctataagtcaatatgaagttag.

32. The kit according to any one of claims 24 - 31 wherein said kit comprises oligonucleotides selected from the group comprising:
tactttaatactaacac; tttaatactaacactgc; actaacactgctgttgc; tgctgttgcagttactg; tgctgttgcagttactggatgg; tgttgcagttactggatggcaa; atggcaaactattaatggtaaa; gatggcaaactattaatggtaa.

33. The kit according to any one of claims 24 - 31 wherein said kit comprises oligonucleotides selected from the group comprising:
ctgcgaactattgatgg; atggtaaaaaatattac; aaatattactttaatac; attactttaatactaac; tactttaatactaacac; gtaaaaaatattactttaatac; aaaatattactttaatactaac; aatattactttaatactaacac; attactttaatactaacactgc; tactttaatactaacactgctg; ttaatactaacactgctgttgc; and aatactaacactgctgttgcag

34. An array comprising any at least one oligonucleotides probe according to claims 1-33.

## Patentansprüche

1. Verfahren zum Nachweis von C. difficile in einer Probe, wobei das C. difficile eine Nukleinsäurezielregion mit der folgenden Sequenz umfasst:
I) Tcaagactctattatagtaagtgcaaatcaatatgaagt;
wobei das Verfahren das Aussetzen der Probe gegenüber Folgendem umfasst:
a) wenigstens einer Oligonukleotidsonde, die wenigstens zu 15 Nukleotiden der Zielregion komplementär ist, oder;
b) einer Oligonukleotidsonde, die wenigstens zu 80 % homolog zu dem Oligonukleotid aus a) ist; und
das Nachweisen der Bindung des Oligonukleotids an die Zielregion und, wenn eine Bindung auftritt, Schlussfolgern, dass C. difficile in der Probe vorliegt.

2. Verfahren nach Anspruch 1, wobei nacheinander oder gleichzeitig mit dem Verfahren aus Anspruch 1 ein weiterer Test an der Probe durchgeführt wird, um zu bestimmen, ob eine weitere Nukleinsäurezielregion mit der folgenden Sequenz vorliegt:
II) Aaaatattactttaatactaacactgctgttgcagttactggatggcaaactattaa tggtaaaaaatactacttt;
wobei das Verfahren das Aussetzen der Probe gegenüber Folgendem umfasst:
d) wenigstens einer Oligonukleotidsonde, die wenigstens zu einem Teil der weiteren Nukleinsäurezielregion komplementär ist, oder;
e) einer Oligonukleotidsonde, die wenigstens zu 80 % homolog zu den Oligonukleotiden aus d) ist; und
das Nachweisen der Bindung des Oligonukleotids an die weitere Nukleinsäurezielregion und, wenn eine Bindung auftritt, Schlussfolgern, dass C. difficile in der Probe vorliegt.

3. Verfahren nach einem der vorhergehenden Ansprüche, wobei die Probe ausgewählt ist aus der Gruppe umfassend: Erde; Boden; Fäkalien; Urin; Körperflüssigkeit; Lebensmittel; Laborkulturen; Krankenhausausrüstung oder Wundverbände.

4. Verfahren nach einem der vorhergehenden Ansprüche, wobei DNA aus der Probe extrahiert wird.

5. Verfahren nach einem der vorhergehenden Ansprüche, wobei das Oligonukleotid zur Verwendung in einem Oligonukleotid-Bindungsassay bestimmt ist, das ausgewählt ist aus der Gruppe, umfassend: PCR, Southern Blotting, DNA-Dot-Blotting, DNA-Microarray-Techniken, mikrowellenunterstützte Annealing-Assays.

6. Verfahren nach einem der vorhergehenden Ansprüche, wobei das Oligonukleotid Folgendes umfasst:
ein Signalmolekül, das ein Signal abgibt, wenn das Oligonukleotid entweder in gebundenem oder ungebundenem Zustand vorliegt; oder ein quantitatives Signal abgibt, dessen Skala für wenigstens einen des gebundenen oder ungebundenen Zustands repräsentativ ist.

7. Verfahren nach einem der vorhergehenden Ansprüche, wobei das Oligonukleotid einen Teil eines Signalgebungssystems umfasst, wobei der Teil mit anderen Teilen des Systems interagiert, um ein Signal abzugeben, wenn es sich entweder in einem gebunden Zustand oder einem ungebundenen Zustand befindet, wodurch die Bindung des Oligonukleotids an die Zielregion nachgewiesen werden kann.

8. Verfahren nach einem der Ansprüche 6-7, wobei das Signalmolekül ausgewählt sein kann aus der Gruppe, umfassend: ein fluoreszierendes Molekül; ein chemilumineszierendes Molekül oder ein biolumineszierendes Molekül.

9. Verfahren nach einem der vorhergehenden Ansprüche, wobei die Oligonukleotidsonde ein Sondenpaar umfasst, einschließlich einer Ankersonde und einer markierten Nachweissonde.

10. Verfahren nach Anspruch 9, wobei die Ankersonde eine Stelle bindet, die zwischen 1-20 Nukleotide von der durch die Nachweissonde gebundenen Stelle getrennt ist.

11. Verfahren nach Anspruch 9 oder 10 wobei die Ankersonde eine Stelle bindet, die 5 Nukleotide von der durch die Nachweissonde gebundenen Stelle getrennt ist.

12. Verfahren nach einem der Ansprüche 9-11, wobei die Ankersonde ferner eine chemische Gruppe/ein Molekül zum Befestigen oder Anhaften der Sonde an einer Oberfläche umfasst.

13. Verfahren nach Anspruch 12, wobei die chemische Gruppe/das Molekül eine Thiolgruppe oder Biotin ist.

14. Verfahren nach Anspruch 12 oder 13, wobei die Ankersonde ferner wenigstens eine T-Nukleotidbase zwischen der chemischen Gruppe/dem Molekül und einem Bindungsnukleotid der Ankersonde an der Zielregion umfasst.

15. Verfahren nach einem der vorhergehenden Ansprüche, umfassend eine Vielzahl von Oligonukleotidsonden, die mit den gleichen oder unterschiedlichen Signalmolekülen markiert sind.

16. Verfahren nach einem der vorhergehenden Ansprüche, wobei die Konzentration der Oligonukleotidsonden zwischen 10-100 ng/ul liegt.

17. Verfahren nach einem der vorhergehenden Ansprüche, wobei die Proben ausgewählt sind aus der Gruppe, umfassend:
eine Länge zwischen 15-30 Nukleotiden oder 15-25 Nukleotiden oder 17-22 Nukleotiden.

18. Verfahren nach einem der vorhergehenden Ansprüche, wobei das/die Oligonukleotid (e) ausgewählt ist/sind aus der Gruppe, umfassend:
tcaagactctattatag und taagtgcaaatcaatatgaagt.

19. Verfahren nach einem der vorhergehenden Ansprüche, wobei das Oligonukleotid Folgendes ist:
ctataagtcaatatgaagttag

20. Verfahren nach einem der Ansprüche 2-19, wobei die Oligonukleotide ausgewählt sind aus der Gruppe, umfassend:
tactttaatactaacac; tttaatactaacactgc; actaacactgctgttgc; tgctgttgcagttactg; tgctgttgcagttactggatgg; tgttgcagttactggatggcaa; atggcaaactattaatggtaaa und gatggcaaactattaatggtaa.

21. Verfahren nach einem der Ansprüche 2-19, wobei die Oligonukleotide ausgewählt sind aus der Gruppe, umfassend:
ctgcgaactattgatgg; atggtaaaaaatattac; aaatattactttaatac; attactttaatactaac; tactttaatactaacac; gtaaaaaatattactttaatac; aaaatattactttaatactaac; aatattactttaatactaacac; attactttaatactaacactgc; tactttaatactaacactgctg; ttaatactaacactgctgttgc und aatactaacactgctgttgcag

22. Verfahren nach einem der vorhergehenden Ansprüche, wobei das Verfahren ein Diagnoseverfahren zum Identifizieren von Individuen ist, die an einer Infektion mit C. difficile leiden.

23. Kit zum Nachweis von C. difficile in einer Probe, wobei das C. difficile eine Nukleinsäurezielregion mit der folgenden Sequenz umfasst:
I) Tcaagactctattatagtaagtgcaaatcaatatgaagt;
wobei das Kit Folgendes umfasst:
a) wenigstens eine Oligonukleotidsonde, die wenigstens zu 15 Nukleotiden der Zielregion komplementär ist; oder;
b) eine Oligonukleotidsonde, die wenigstens zu 84 % homolog zu dem Oligonukleotid aus a) ist; und
optional Reagenzien und/oder eine Anleitung zum Ausführen des Nachweises.

24. Kit nach Anspruch 23, wobei das C. difficile ferner eine Nukleinsäurezielregion mit der folgenden Sequenz umfasst:
II) Aaaatattactttaatactaacactgctgttgcagttactggatggcaaactattaa tggtaaaaaatactacttt;
und wobei das Kit ferner Folgendes umfasst:
d) wenigstens eine Oligonukleotidsonde, die wenigstens zu einem Teil der weiteren Nukleinsäurezielregion komplementär ist, oder;
e) eine Oligonukleotidsonde, die wenigstens zu 80 % homolog zu den Oligonukleotiden aus d) ist; und
das Nachweisen der Bindung des Oligonukleotids an die weitere Nukleinsäurezielregion und, wenn eine Bindung auftritt, Schlussfolgern, dass C. difficile in der Probe vorliegt.

25. Kit nach Anspruch 23 oder 24, wobei das Oligonukleotid Folgendes umfasst:
ein Signalmolekül, das ein Signal abgibt, wenn das Oligonukleotid entweder in gebundenem oder ungebundenem Zustand vorliegt; oder ein quantitatives Signal abgibt, dessen Skala für wenigstens einen des Zustände repräsentativ ist.

26. Kit nach einem der Ansprüche 23-25, wobei das Oligonukleotid einen Teil eines Signalgebungssystems umfasst, wobei der Teil mit anderen Teilen des Systems interagiert, um ein Signal abzugeben, wenn es sich entweder in einem gebunden Zustand oder einem ungebundenen Zustand befindet, wodurch die Bindung des Oligonukleotids an die Zielstelle nachgewiesen werden kann.

27. Kit nach einem der Ansprüche 23-26, wobei die Oligonukleotidsonde ein Sondenpaar umfasst, einschließlich einer Ankersonde und einer markierten Nachweissonde.

28. Kit nach Anspruch 27, wobei die Ankersonde ferner eine chemische Gruppe/ein Molekül zum Befestigen oder Anhaften der Sonde an einer ausgewählten Oberfläche umfasst.

29. Kit nach Anspruch 28, wobei die Ankersonde ferner wenigstens eine T-Nukleotidbase zwischen der chemischen Gruppe/dem Molekül und einem Bindungsnukleotid der Ankersonde an der Zielregion umfasst.

30. Kit nach einem der Ansprüche 23-29, wobei das Kit wenigstens ein Oligonukleotid umfasst, ausgewählt aus der Gruppe, umfassend:
tcaagactctattatag und taagtgcaaatcaatatgaagt.

31. Kit nach einem der Ansprüche 23-29, wobei das Kit wenigstens ein Oligonukleotid umfasst, das Folgendes ist:
ctataagtcaatatgaagttag.

32. Kit nach einem der Ansprüche 24-31, wobei das Kit Oligonukleotide umfasst, die ausgewählt sind aus der Gruppe, umfassend:
tactttaatactaacac; tttaatactaacactgc; actaacactgctgttgc; tgctgttgcagttactg; tgctgttgcagttactggatgg; tgttgcagttactggatggcaa; atggcaaactattaatggtaaa; gatggcaaactattaatggtaa.

33. Kit nach einem der Ansprüche 24-31, wobei das Kit Oligonukleotide umfasst, die ausgewählt sind aus der Gruppe, umfassend:
ctgcgaactattgatgg; atggtaaaaaatattac; aaatattactttaatac; attactttaatactaac; tactttaatactaacac; gtaaaaaatattactttaatac; aaaatattactttaatactaac; aatattactttaatactaacac; attactttaatactaacactgc; tactttaatactaacactgctg; ttaatactaacactgctgttgc und aatactaacactgctgttgcag

34. Array, umfassend wenigstens eine Oligonukleotidsonde nach den Ansprüchen 1-33.

## Revendications

1. Procédé pour la détection de *C. difficile* dans un échantillon dans lequel ledit *C. difficile* comprend une région cible d'acide nucléique ayant la séquence suivante :
I) Tcaagactctattatagtaagtgcaaatcaatatgaagt ;
lequel procédé comprend l'exposition dudit échantillon à :
a) au moins une sonde oligonucléotidique qui est complémentaire d'au moins 15 nucléotides de ladite région cible, ou ;
b) une sonde oligonucléotidique qui est homologue à au moins 80 % à l'oligonucléotide en a) ; et
la détection de la liaison dudit oligonucléotide à ladite région cible et, lorsqu'une liaison survient, la conclusion que *C. difficile* est présent dans ledit échantillon.

2. Procédé selon la revendication 1 dans lequel un autre test est entrepris sur ledit échantillon, séquentiellement ou simultanément, au procédé selon la revendication 1 pour déterminer si une autre région cible d'acide nucléique ayant la séquence suivante est présente :
II) Aaaatattactttaatactaacactgctgttgcagttactggatggcaaactattaat ggtaaaaaatactacttt ;
lequel procédé comprend l'exposition dudit échantillon à :
d) au moins une sonde oligonucléotidique qui est complémentaire d'au moins une partie de ladite autre région cible d'acide nucléique, ou ;
e) une sonde oligonucléotidique qui est homologue à au moins 80 % aux oligonucléotides en d) ; et
la détection de la liaison dudit oligonucléotide à ladite autre région cible d'acide nucléique et, lorsqu'une liaison survient, la conclusion que *C. difficile* est présent dans ledit échantillon.

3. Procédé selon l'une quelconque des revendications précédentes dans lequel ledit échantillon est choisi dans le groupe comprenant : de la terre ; du sol ; des selles ; de l'urine ; un liquide corporel ; un aliment ; des cultures de laboratoire ; un équipement hospitalier ; ou des pansements.

4. Procédé selon l'une quelconque des revendications précédentes dans lequel l'ADN est extrait dudit échantillon.

5. Procédé selon l'une quelconque des revendications précédentes dans lequel ledit oligonucléotide est conçu pour une utilisation dans un test de liaison d'oligonucléotide choisi dans le groupe comprenant : la PCR, l'analyse Southern blot, l'analyse Dot blot d'ADN, les techniques de micropuces d'ADN, les techniques d'hybridation assistée par micro-ondes.

6. Procédé selon l'une quelconque des revendications précédentes dans lequel ledit oligonucléotide comprend :
une molécule de signalisation qui émet un signal lorsque ledit oligonucléotide existe dans un état soit lié soit non lié ; ou émet un signal quantitatif dont l'échelle est représentative d'au moins l'un desdits états lié ou non lié.

7. Procédé selon l'une quelconque des revendications précédentes dans lequel ledit oligonucléotide comprend une partie d'un système de signalisation, laquelle partie interagit avec d'autres parties dudit système pour émettre un signal lorsqu'il est soit dans un état lié soit dans un état non lié, moyennant quoi la liaison dudit oligonucléotide à ladite région cible peut être détectée.

8. Procédé selon l'une quelconque des revendications 6 ou 7 dans lequel ladite molécule de signalisation peut être choisie dans le groupe comprenant : une molécule fluorescente ; une molécule chimioluminescente ; ou une molécule bioluminescente.

9. Procédé selon l'une quelconque des revendications précédentes dans lequel ladite sonde oligonucléotidique comprend une paire de sondes incluant une sonde d'ancrage et une sonde détecteur marquée.

10. Procédé selon la revendication 9 dans lequel ladite sonde d'ancrage lie un site séparé d'entre 1 à 20 nucléotides du site lié par ladite sonde détecteur.

11. Procédé selon la revendication 9 ou 10 dans lequel ladite sonde d'ancrage lie un site séparé de 5 nucléotides du site lié par ladite sonde détecteur.

12. Procédé selon l'une quelconque des revendications 9 à 11 dans lequel ladite sonde d'ancrage comprend en outre un groupe/une molécule chimique pour la fixation, ou l'adhérence, de ladite sonde à une surface.

13. Procédé selon la revendication 12 dans lequel ledit groupe/ladite molécule chimique est un groupe thiol ou la biotine.

14. Procédé selon la revendication 12 ou 13 dans lequel ladite sonde d'ancrage comprend en outre au moins une base nucléotidique T entre ledit groupe/ladite molécule chimique et un nucléotide de liaison de ladite sonde d'ancrage à ladite région cible.

15. Procédé selon l'une quelconque des revendications précédentes comprenant une pluralité de sondes oligonucléotidiques marquées avec des molécules de signalisation identiques ou différentes.

16. Procédé selon l'une quelconque des revendications précédentes dans lequel la concentration desdites sondes oligonucléotidiques se situe entre 10 et 100 ng/µl.

17. Procédé selon l'une quelconque des revendications précédentes dans lequel lesdites sondes sont choisies dans le groupe comprenant :
entre 15 et 30 nucléotides ou entre 15 et 25 nucléotides ou entre 17 et 22 nucléotides en longueur.

18. Procédé selon l'une quelconque des revendications précédentes dans lequel le(s)dit(s) oligonucléotide(s) est/sont choisi(s) dans le groupe comprenant :
tcaagactctattatag ; et taagtgcaaatcaatatgaagt.

19. Procédé selon l'une quelconque des revendications précédentes dans lequel ledit oligonucléotide est :
ctataagtcaatatgaagttag.

20. Procédé selon l'une quelconque des revendications 2 à 19 dans lequel lesdits oligonucléotides sont choisis dans le groupe comprenant :
tactttaatactaacac ; tttaatactaacactgc ; actaacactgctgttgc ; tgctgttgcagttactg ; tgctgttgcagttactggatgg ; tgttgcagttactggatggcaa ; atggcaaactattaatggtaaa ; et gatggcaaactattaatggtaa.

21. Procédé selon l'une quelconque des revendications 2 à 19 dans lequel lesdits oligonucléotides sont choisis dans le groupe comprenant :
ctgcgaactattgatgg ; atggtaaaaaatattac ; aaatattactttaatac ; attactttaatactaac ; tactttaatactaacac ; gtaaaaaatattactttaatac ; aaaatattactttaatactaac ; aatattactttaatactaacac ; attactttaatactaacactgc ; tactttaatactaacactgctg ; ttaatactaacactgctgttgc ; et aatactaacactgctgttgcag.

22. Procédé selon l'une quelconque des revendications précédentes dans lequel ledit procédé est un procédé de diagnostic pour l'identification d'individus souffrant d'une infection à *C. difficile.*

23. Kit pour la détection de *C. difficile* dans un échantillon dans lequel ledit *C. difficile* comprend une région cible d'acide nucléique ayant la séquence suivante :
I) Tcaagactctattatagtaagtgcaaatcaatatgaagt ;
lequel kit comprend :
a) au moins une sonde oligonucléotidique qui est complémentaire d'au moins 15 nucléotides de ladite région cible, ou ;
b) une sonde oligonucléotidique qui est homologue à au moins 84 % à l'oligonucléotide en a) ; et
éventuellement, des réactifs et/ou des instructions pour pratiquer ladite détection.

24. Kit selon la revendication 23 dans lequel ledit *C. difficile* comprend une région cible d'acide nucléique ayant la séquence suivante :
II) Aaaatattactttaatactaacactgctgttgcagttactggatggcaaactattaat ggtaaaaaatactacttt ;
et lequel kit comprend en outre :
d) au moins une sonde oligonucléotidique qui est complémentaire d'au moins une partie de ladite autre région cible d'acide nucléique, ou ;
e) une sonde oligonucléotidique qui est homologue à au moins 80 % aux oligonucléotides en d) ; et
la détection de la liaison dudit oligonucléotide à ladite autre région cible d'acide nucléique et, lorsqu'une liaison survient, la conclusion que *C. difficile* est présent dans ledit échantillon.

25. Kit selon la revendication 23 ou 24 dans lequel ledit oligonucléotide comprend :
une molécule de signalisation qui émet un signal lorsque ledit oligonucléotide existe dans un état soit lié soit non lié ; ou émet un signal quantitatif dont l'échelle est représentative d'au moins l'un desdits états.

26. Kit selon l'une quelconque des revendications 23 à 25 dans lequel ledit oligonucléotide comprend une partie d'un système de signalisation, laquelle partie interagit avec d'autres parties dudit système pour émettre un signal lorsqu'il est soit dans un état lié soit dans un état non lié, moyennant quoi la liaison dudit oligonucléotide au dit site cible peut être détectée.

27. Kit selon l'une quelconque des revendications 23 à 26 dans lequel ladite sonde oligonucléotidique comprend une paire de sondes incluant une sonde d'ancrage et une sonde détecteur marquée.

28. Kit selon la revendication 27 dans lequel ladite sonde d'ancrage comprend en outre un groupe/une molécule chimique pour la fixation, ou l'adhérence, de ladite sonde à une surface choisie.

29. Kit selon la revendication 28 dans lequel ladite sonde d'ancrage comprend en outre au moins une base nucléotidique T entre ledit groupe/ladite molécule chimique et un nucléotide de liaison de ladite sonde d'ancrage à ladite région cible.

30. Kit selon l'une quelconque des revendications 23 à 29 dans lequel ledit kit comprend au moins un oligonucléotide choisi dans le groupe comprenant :
tcaagactctattatag ; et taagtgcaaatcaatatgaagt.

31. Kit selon l'une quelconque des revendications 23 à 29 dans lequel ledit kit comprend au moins un oligonucléotide qui est :
ctataagtcaatatgaagttag.

32. Kit selon l'une quelconque des revendications 24 à 31 dans lequel ledit kit comprend des oligonucléotides choisis dans le groupe comprenant :
tactttaatactaacac ; tttaatactaacactgc ; actaacactgctgttgc ; tgctgttgcagttactg ; tgctgttgcagttactggatgg ; tgttgcagttactggatggcaa ; atggcaaactattaatggtaaa ; et gatggcaaactattaatggtaa.

33. Procédé selon l'une quelconque des revendications 24 à 31 dans lequel ledit kit comprend des oligonucléotides choisis dans le groupe comprenant :
ctgcgaactattgatgg ; atggtaaaaaatattac ; aaatattactttaatac ; attactttaatactaac ; tactttaatactaacac ; gtaaaaaatattactttaatac ; aaaatattactttaatactaac ; aatattactttaatactaacac ; attactttaatactaacactgc ; tactttaatactaacactgctg ; ttaatactaacactgctgttgc ; et aatactaacactgctgttgcag.

34. Puce comprenant au moins une sonde oligonucléotidique quelconque selon les revendications 1 à 33.
